# EUROPEAN PATENT SPECIFICATION

(11) **EP 1 196 444 B1**
(45) Date of publication and mention of the grant of the patent: **04.06.2003**
(21) Application number: 00945656.7
(22) Date of filing: 12.07.2000
(51) Int. Cl.: C07K 14/575, A61K 38/22, A61K 47/48, A61P 3/10

(54) **EXTENDIN-4 CONJUGATES AND THEIR MEDICAL USE**
EXTENDIN-4 KONJUGATE UND IHRE MEDIZINISCHE VERWENDUNG
CONJUGATES D'EXTENDIN-4 ET LEUR UTILISATION MEDICALE

(30) Priority: 12.07.1999 US 143591 P; 09.08.1999 EP 99610043
(43) Date of publication of application: 17.04.2002
(60) Divisional application: 03005786.3
(73) Proprietor: Zealand Pharma A/S, 2600 Glostrup (DK)
(72) Inventor: LARSEN, Bjarne, Due, DK-2700 Bronshoj (DK); MIKKELSEN, Jens, Damsgaard, DK-2800 Lyngby (DK); NEVE, Soren, DK-2800 Lyngby (DK)
(74) Representative: Kiddle, Simon John
(86) International application number: DK0000393
(87) International publication number: WO01004156

(56) References cited:
- WO-A-98/05351
- WO-A-98/08871
- WO-A-98/11126
- WO-A-98/22577
- WO-A-98/30231
- WO-A-99/07404
- WO-A-99/25727
- WO-A-99/25728
- WO-A-99/43708
- WO-A-99/46283
- MEURER JANET A ET AL: "Properties of native and in vitro glycosylated forms of the glucagon-like peptide-1 receptor antagonist exendin (9-39)" BIOSIS, XP002146590

## Description

### FIELD OF THE INVENTION

The present invention relates to peptide conjugates of exendin-4, and especially conjugates that lower blood glucose levels. The conjugates may be used in the treament of diseases that benefit from regulation of excess levels of blood glucose and/or regulation of gastric emptying, such as diabetes and eating disorders.

### BACKGROUND OF THE INVENTION

A number of hormones that lower blood glucose levels are released from the gastrointestinal mucosa in response to the presence and absorption of nutrients in the gut. These include gastrin, secretin, glucose-dependent insulinotropic polypeptide (GIP) and glucagon-like peptide-1 (GLP-1). The most potent substance known is GLP-1 (Ørskov, 1992, Diabetologia 35:701-711). Glucagon-like peptide 1 (GLP-1) is a product of proglucagon, a 180 amino acid peptide (Drucker, 1998, Diabetes 47:159-169). The overall sequence of proglucagon contains the 29-amino acid sequence of glucagon, the 36 or 37 amino acid sequence of GLP-1 and the 34 amino acid sequence of glucagon-like peptide-2 (GLP-2), an intestinotrophic peptide. GLP-1 has a number of functions. It is a physiological hormone that enhances the effect on insulin secretion in normal humans and is therefore an incretin hormone. In addition, GLP-1 also lowers glucagon concentrations, slows gastric emptying, stimulates (pro)insulin biosynthesis, and enhances insulin sensitivity (Nauck, 1997, Horm. Metab. Res. 47:1253-1258). The peptide also enhances the ability for the β-cells to sense and respond to glucose in subjects with impaired glucose tolerance (Byrne, 1998, Eur. J. Clin. Invest. 28:72-78). The insulinotropic effect of GLP-1 in humans increases the rate of glucose disappearance partly because of increased insulin levels and partly because of enhanced insulin sensitivity (D'Alessio, 1994, Eur. J. Clin. Invest. 28:72-78). This has placed GLP-1 as a promising agent for treatment for type II diabetes. Active fragments of GLP-1 have been found to be GLP-1(7-36) and GLP-1(7-37). However, a major pharmacological problem with native GLP-1 is its short half-life. In humans and rats, GLP-1 is rapidly degraded by dipeptidyl peptidase-IV (DPP-IV) into GLP-1(9-36)amide, acting as an endogenous GLP-1 receptor antagonist (Deacon, 1998, Diabetologia 41:271-278). Several strategies circumventing this problem have been proposed, some using inhibitors of DPP-IV and others DPP-IV resistant analogues of GLP-1(7-36)amide (Deacon, 1998, Diabetologia 41:271-278; Deacon et al., 1998, Diabetes 47:764-769; Ritzel, 1998, J. Endocrinol. 159:93-102; U.S. Patent No. 5,545,618; Pederson, 1998, Diabetes 47:1253-1258).

Exendins, another group of peptides that lower blood glucose levels have some sequence similarity (53%) to GLP-1[7-36]NH₂ (Goke et al., 1993, J. Biol. Chem. 268:19650-55). The exendins are found in the venom of Helodermatidae or beaded lizards (Raufman, 1996, Reg. Peptides 61:1-18). Exendin-3 is present in the venom of *Heloderma horridum,* the Mexican beaded lizard and exendin-4 is present in the venom of *Heloderma suspectum,* the Gila monster. Exendin-4 differs from exendin-3 at just positions two and three. The cDNA encoding the exendin-4 precursor protein, a 47 amino acid peptide fused to the amino terminus of exendin-4 has been cloned and sequenced (Pohl et al., 1998, J. Biol. Chem. 273:9778-9784 and WO98/35033). Both exendin-3 and exendin-4 stimulate an increase in cellular cAMP production in guinea pig pancreatic acinar cells by interacting with exendin receptors (Raufman, 1996, Reg. Peptides 61:1-18). Exendin-3 causes a biphasic increase in cellular cAMP production, but a monophasic increase in amylase release in pancreatic acinar cells. In contrast, exendin-4 causes a monophasic increase in cAMP production and does not alter amylase release.

Exendin-4 is a strong GLP-1 receptor agonist on isolated rat insulinoma cells (Goke et al., 1993, J. Biol. Chem. 268:19650-55). This is expected as the (His Ala) domain of GLP-1 recognised by DPP-IV is not present in exendin-4 (Goke et al., 1993, J. Biol. Chem. 268:19650-55). Binding of [¹²⁵I]GLP-1 to the nucleus of the solitary tract was inhibited concentration-dependently by unlabelled GLP-1 and [Tyr39]exendin-4 with Ki values of 3.5 and 9.4 nM respectively, and similar values are found in cell lines (Goke et al., 1995, Eur. J. Neurosci. 7:2294-2300 and Goke et al., 1993, J. Biol. Chem. 268:19650-55). Further, exendin-4 given systemically lowers blood glucose levels by 40% in diabetic db/db mice (WO99/07404). Recently, Grieg et al. (1999, Diabetologia 42:45-50) has shown a long lasting blood glucose lowering effect of once daily intraperitoneal injection of exendin-4 to diabetic *ob*/*ob* mice). US patent No. 5,424,286 discloses that a considerable portion of the N-terminal sequence is essential in order to preserve insulinotropic activity (exendin4(1-31) and Y³¹ -exendin-4(1-31)) whereas an N-terminally truncated exendin (exendin-4(9-39) has inhibitory properties.

The use of exendin-3, exendin-4 and exendin agonists has been proposed for the treatment of diabetes mellitus, reducing gastric motility and delaying gastric emptying and the prevention of hyperglycemia (U.S. Patent No. 5,424,286, WO98/05351) as well as for the reduction of food intake (WO98/30231). There has been proposed ways of obtaining novel compounds by modifying the native exendin sequences. One way is to attach lipophilic substituents to the molecule, e.g. as described in WO 99/43708 which discloses derivatives of exendin with just one lipophilic substituent attached to the C-terminal amino acid residue.

A major approach has been to devise exendin analogues characterised by amino acid substitutions and/or C-terminal truncation of the native exendin-4 sequence. This approach is represented by the compounds of WO99/07404, WO 99/25727 and WO 99/25728.
WO99/07404 discloses exendin agonists having a general formula I that defines a peptide sequence of 39 amino acid residues with Gly Thr in positions 4-5, Ser Lys Gln in positions 11-13, Glu Glu Glu Ala Val Arg Leu in positions 15-21, Leu Lys Asn Gly Gly in positions 26-30, Ser Ser Gly Ala in positions 32-35, and wherein the remaining positions may be occupied by wild-type exendin amino acid residues or may be occupied by specified amino acid substitutions. The formula I does not cover any exendin agonists or analogues having specific amino acid deletions and/or being conjugates as described herein, such as the novel compounds desPro³⁶-exendin4(1-39), exendin4(1-39)-K₆ or desPro³⁶-exendin-4( 1-39)-K₆.
WO 99/25727 discloses exendin agonists having a general formula I that defines a peptide sequence of from 28 to 38 amino acid residues with Gly in position 4 and Ala in position 18, and wherein the remaining positions may be occupied by wild-type exendin amino acid residues or may be occupied by specified amino acid substitutions. Formula I does not comprise a peptide sequence having Ser as the C-terminal amino acid and exendin agonists or analogues having specific amino acid deletions and/or being conjugates as described herein, such as the novel compounds desPro³⁶-exendin-4(1-39), exendin-4(1-39)-K₆ or desPro³⁶-exendin4(1-39)-K₆. Further, formula II of WO 99/25727 defines a peptide sequence similar to formula I, but including exendin derivatives having a C(1-10)alkanoyl or cycloalkylalkanoyl substituent on lysine in position 27 or 28.
When treating inappropriate post-prandial blood glucose levels the compounds are administered frequently, for example one, two or three times a day.
WO 99/25728 discloses exendin agonists having a general formula I that defines a peptide sequence of from 28 to 39 amino acid residues with fixed Ala in position 18, and wherein the remaining positions may be occupied by wild-type exendin amino acid residues or may be occupied by specified amino acid substitutions. Said exendin agonists all correspond to a truncated exendin analogue having a varying degree of amino acid substitutions. Peptide sequences of from 34 to 38 amino acid residues do not have Ser C-terminally. A peptide sequence of 39 amino acid residues may have either Ser or Tyr C-terminally, but no further residues. Exendin agonists or analogues having specific amino acid deletions and/or being conjugates according to the invention described herein are not comprised by formula I. Further, formula II defines a peptide sequence similar to formula I, but including exendin derivatives having a C(1-10)alkanoyl or cycloalkylalkanoyl substituent on lysine in position 27 or 28.

WO 99/46283 (published 16.09.99) discloses peptide conjugates comprising a pharmacologically active peptide X and a stabilising peptide sequence Z of 4-20 amino acid residues covalently bound to X, where said conjugates are characterised in having an increased half-life compared to the half-life of X. X may be exendin-4 or exendin-3.

### OBJECTIVE OF THE INVENTION

There is a need for compounds that lower blood glucose levels in mammals, and are stable and effective. Therefore, it is an objective of the invention to provide novel compounds that lower blood glucose levels in mammals. Ideally, these should be effective when administered orally. It is a further object of the invention to provide novel peptide agonists of exendin-4 activity. It is a still further purpose of the invention to provide peptide agonists of exendin-4 activity having an increased half-life and/or a decreased clearance.

### SUMMARY OF THE INVENTION

The invention is directed to a peptide conjugate as set out in claims 1 to 8 and to the medical uses of these conjugates and to pharmaceutical compositions comprising them.

### BRIEF DESCRIPTION OF THE FIGURES

Figure 1 shows the effect of Compound 1 (SEQ ID NO:101) (des Pro³⁶-exendin-4(1-39)-NH₂) on blood glucose levels of mice, cf. Example 15.
Figure 2 shows the effect of Compound 2 (SEQ ID NO:93) (dcs Pro³⁶-exendin-4(1-39)-Lys₆-NH₂ on the blood glucose levels of mice, cf. Example 15.
Figure 3 is a plot of AUC (area under the curve) values (mean±SEM) for Compounds 2, 14-16, 18 and 19 in an oral glucose tolerance test (OGTT), cf. Examples 17.

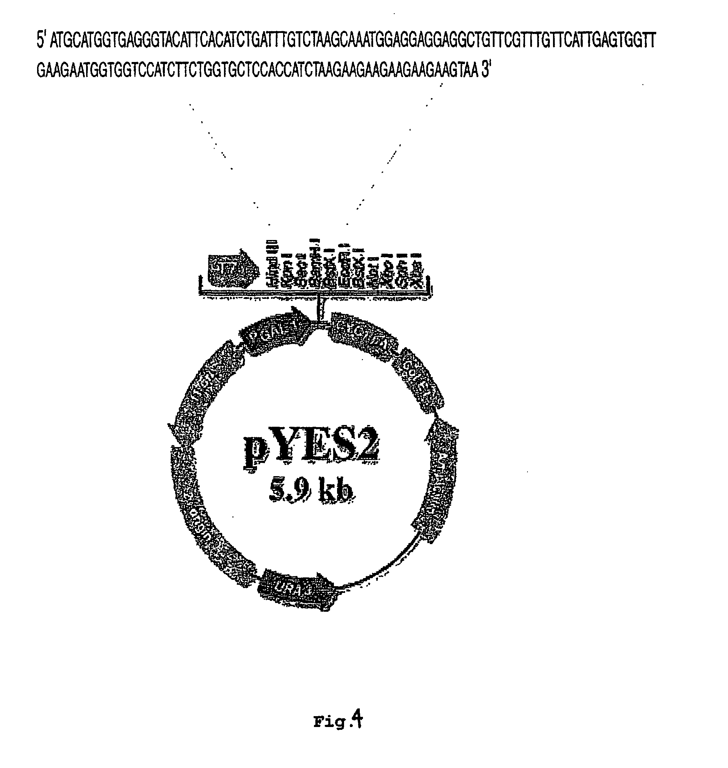

Figure 4 shows a synthetic cDNA constructed for heterolog expression of Compound 2 in yeast. The new construct was designated pYES0010, cf. Example 12.
Figure 5 is a plot of dose-response in a glucose tolerance test (GTT) in db/db mice based on relative AUC₀₋₂₄₀ₘᵢₙ values (mean±SEM) for Compound 2 and Compound (i), cf. Example 18.
Figure 6 shows the effects of a maximal dose of Compound 2, i.e. 100 nmol/kg i.p., on the oral glucose tolerance test (OGTT) when administered up to 24 hours before the OGTT.

### DETAILED DESCRIPTION OF THE INVENTION

The compounds of the present invention include hitherto unknown deletion variants of a parent exendin. In contrast to known substitution and/or truncation variants of exendin-4(1-39) the novel compounds are believed to exhibit a stabilised alpha-helix structure with superior stability properties and unreduced or enhanced binding properties. Moreover, conjugation of the novel variants, to specific short peptide sequences (Z) render stability to these compounds without compromising the pharmacological properties. These conjugations confer in vivo stability and hydrophilicity to the peptide molecule. The Z is composed of amino-acid residues, and has alone no structural characteristics in terms of α-helix conformation. However, from studies using both circular dichroism and nuclear magnetic resonance (NMR) spectroscopy, addition of Z dramatically alters the structural characteristics of some peptides as evidenced by the increased amount of α-helix conformation in the peptide. Together with the pharmacological results, the structural analyses suggest that Z is modifying the conformation of the peptide leading to higher enzyme-stability, but without losing its potency. Also the physical and chemical properties of peptides may be altered considerably by Z-modification with resulting impact on pharmacological formulation strategy.

### Exendin Variants

The exendin variant of the present invention is a variant of a parent exendin peptide comprising between one and five deletions at positions 34-38. Preferably the variant comprises between 1 and 4 deletions at positions 34-38, more preferably between 1 and 3 deletions at positions 36-38. Preferably the parent exendin is exendin-4, and a preferred variant included as peptide X in the peptide conjugates herein has an amino acid sequence wherein 1, 2 or 3 of the Pro residues in positions 36, 37 and 38 have been deleted from the amino acid sequence of exendin-4 and preferably from the amino acid sequence of exendin-4(1-39).

Coupling of a Z sequence to the X peptide herein is believed to increase the stability of these compounds. Proline is a rigid amino acid that may interfere with the effect of Z to stabilise the structure of the X peptide. Deletion of one, two or all of the proline amino acids in positions 36, 37 and 38 of the exendin backbone is therefore preferred in the peptide conjugates comprising a variant of a parent exendin according.to the invention, as long as the efficacy of said conjugates as measured in, e.g. an oral glucose tolerance test (OGTT) in diabetic db/db mice, is not negatively affected.

In another embodiment, the variant comprises an additional residue at position 40, a lysine residue which comprises a lipophilic substituent bound to the epsilon amino group of lysine via an amide bond. The lipophilic substituent may be the acyl group of a straight-chain or branched fatty acid or a straight-chain or branched alkane α,ω-dicarboxylic acid. The acyl group may have the formula CH₃(CH₂)ₙCO-, wherein n is an integer from 4-38 and preferably from 4-24. In a specific embodiment, the acyl group is selected from the group consisting of CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO-, and CH₃(CH₂)₂₂CO. The acyl group may have the formula HOOC(CH₂)ₘCO-, wherein n is an integer from 4-38 and preferably from 4-24. In a specific embodiment, the acyl group is selected from the group consisting of HOOC(CH₂)₁₄CO-, HOOC(CH₂)₁₆CO-, HOOC(CH₂)₁₈CO-, HOOC(CH₂)₂₀CO- and HOOC(CH₂)₂₂CO-. In a more specific embodiment, the lipophilic substituent is selected from the group consisting of tetradecanoyl, ω-carboxynonadecanoyl, 7-deoxycholoyl, choloyl, palmitoyl and lithocholyl. In a most specific embodiment, the lipophilic substituent is palmitoyl.

Alternatively, the liphophilic substituent may have an NH group. Specific embodiments include but are not limited to the formulae CH₃(CH₂)ₐ((CH₂)_{b}COOH)CHNHCO(CH₂)₂CO-wherein a and b are integers and a+b is an integer of from 8 to 33, preferably from 12 to 28;
CH₃(CH₂)_{c}CONHCH(COOH) (CH₂)₂CO- wherein c is an integer of from 10 to 24; CH₃(CH₂)_{d} CONHCH(CH₂)₂ (COOH)CO- wherein d is an integer of from 8 to 24; COOH(CH₂)ₑCO- wherein e is an integer of from 8 to 24;
-NHCH(COOH)(CH₂)₄NHCO(CH₂)_{f}CH₃ wherein f is an integer of from 8 to 18;
- NHCH(COOH)(CH₂)₄NHCOCH(CH₂)₂COOH)NHCO(CH₂)_{g}CH₃ wherein g is an integer of from 10 to 16; and -NHCH(COOH)(CH₂)₄NHCO(CH₂)₂CH(COOH)NHCO(CH₂)ₕCH₃ wherein h is an integer of 0 or from 1 to 22 and preferably from 10 to 16.

The exendin variants having a lysine residue at position 40 carrying a lipophilic substituent optionally further comprise between one and five deletions, preferably between one and three deletions, at positions 34 to 39, preferably at positions 34-38, such as [des Ser³⁹, Lys⁴⁰ (palmitoyl)]exendin-4(1-39), [des Pro³⁶, Lys⁴⁰ (palmitoyl)]exendin-4(1-39) and [des Pro³⁶, Lys⁴⁰ (palmitoyl)]exendin-4(1-40).

The variant may be in a most specific embodiment selected from the group consisting of:
Compound 1: des Pro³⁶-exendin-4(1-39)-NH₂ (SEQ ID NO:101),
des Pro³⁶exendin4(1-40)-NH_{2,}
Compound 14: des Pro³⁶, Pro³⁷, Pro³⁸-exendin4(1-39)-NH_{2,}
des Pro³⁶, Pro³⁷, Pro³⁸ exendin-4(1-40)-NH_{2,}
des Pro³⁶, Pro³⁷-exendin-4(1-39)-NH_{2,}
des Ala³⁵-exendin-4(1-39)-NH₂(SEQ ID NO:105),
des Gly³⁴-exendin-4(1-39)-NH₂ (SEQ ID NO:106),
des Ser³⁹-(Lys⁴⁰ (palmitoyl))exendin-4(1-39)-NH₂ (SEQ ID NO:107),
des Gly³⁴-(Lys⁴⁰ (palmitoyl))exendin-4(1-39)-NH₂ (SEQ ID NO:108),
des Ala³⁵ -(Lys⁴⁰ (palmitoyl))exendin-4(1-39)-NH₂ (SEQ ID NO:109),
des Pro³⁶ -(Lys⁴⁰ (palmitoyl))exendin-4(1-39)-NH₂ (SEQ ID NO:110), and the free acid thereof and a pharmaceutically acceptable salt thereof.

### Peptide conjugates

The peptide sequence Z may be bound to the C-terminus of the extendin peptide sequence, preferably via a peptide bond.

Z is a peptide sequence comprising between 1 and 7 Lys residues and is preferably between 4-20 amino acid residues, e.g., in the range of 4-15, more preferably in the range of 4-10 in particular in the range of 4-7 amino acid residues e.g., of 4,5, 6, 7, 8 or 10 amino acid residues, where 6 amino acid residues are preferred. Apart from one or more Lys residues, the other amino acid residues in the peptide sequence Z are independently selected from the group sonsisting of Ala, Leu, Ser, Thr, Tyr, Asn, Gln, Asp, Glu, Lys, Arg, His, Met, Orn, diaminobutanoic acid and diaminopropanoic acid. Preferably, the amino acid residues are selected from Glu, Lys, and Met, especially Lys, or the amino acid residues are selected from the group consisting of Asn, Glu and Lys. The above-mentioned amino acids may have either D- or L-configuration, but preferably the above-mentioned amino acids have an L-configuration.

Thus, illustrative examples of the peptide sequence Z are:
Lys-Lys-Lys-Lys (SEQ ID NO:1), Xaa-Lys-Lys-Lys, Lys-Xaa-Lys-Lys, Lys-Lys-Xaa-Lys, Lys-Lys-Lys-Xaa, Xaa-Xaa-Lys-Lys, Xaa-Lys-Xaa-Lys, Xaa-Lys-Lys-Xaa, Lys-Xaa-Xaa-Lys, Lys-Xaa-Lys-Xaa, Lys-Lys-Xaa-Xaa, Xaa-Xaa-Xaa-Lys, Xaa-Xaa-Lys-Xaa, Xaa-Lys-Xaa-Xaa, Lys-Xaa-Xaa-Xaa, Lys-Lys-Lys-Lys-Lys (SEQ ID NO:3), Xaa-Lys.Lys-Lys-Lys (SEQ ID NO:4), Lys-Xaa-Lys-Lys-Lys (SEQ ID NO:5), Lys-Lys-Xaa-Lys-Lys (SEQ ID NO:6), Lys-Lys-Lys-Xaa-Lys (SEQ ID NO:7), Lys-Lys-Lys-Lys-Xaa, Xaa-Xaa-Lys-Lys-Lys, Xaa-Lys-Xaa-Lys-Lys, Xaa-Lys-Lys-Xaa-Lys, Xaa-Lys-Lys-Lys-Xaa, Lys-Xaa-Xaa-Lys-Lys, Lys-Xaa-Lys-Xaa-Lys, Lys-Xaa-Lys-Lys-Xaa, Lys-Lys-Xaa-Xaa-Lys, Lys-Lys-Xaa-Lys-Xaa, Lys-Lys-Lys-Xaa-Xaa, Lys-Lys-Xaa-Xaa-Xaa, Lys-Xaa-Lys-Xaa-Xaa, Lys-Xaa-Xaa-Lys-Xaa, Lys-Xaa-Xaa-Xaa-Lys, Xaa-Lys-Lys-Xaa-Xaa, Xaa-Lys-Xaa-Xaa-Lys, Xaa-Xaa-Lys-Lys-Xaa, Xaa-Xaa-Lys-Xaa-Lys, Xaa-Xaa-Xaa-Lys-Lys, Lys-Xaa-Xaa-Xaa-Xaa, Xaa-Lys-Xaa-Xaa-Xaa, Xaa-Xaa-Lys-Xaa-Xaa, Xaa-Xaa-Xaa-Lys-Xaa, Xaa-Xaa-Xaa-Xaa-Lys, Lys-Lys-Lys-Lys-Lys-Lys (SEQ ID NO:9), Xaa-Lys-Lys-Lys-Lys-Lys (SEQ ID NO:10), Lys-Xaa-Lys-Lys-Lys-Lys (SEQ ID NO:11), Lys-Lys-Xaa-Lys-Lys-Lys (SEQ ID NO:12), Lys-Lys-Lys-Xaa-Lys-Lys (SEQ ID NO:13), Lys-Lys-Lys-Lys-Xaa-Lys (SEQ ID NO:14), Lys-Lys-Lys-Lys-Lys-Xaa (SEQ ID NO:15), Xaa-Xaa-Lys-Lys-Lys-Lys (SEQ ID NO:16), Xaa-Lys-Xaa-Lys-Lys-Lys (SEQ ID NO:17), Xaa-Lys-Lys-Xaa-Lys-Lys (SEQ ID NO:18), Xaa-Lys-Lys-Lys-Xaa-Lys (SEQ ID NO:19), Xaa-Lys-Lys-Lys-Lys-Xaa (SEQ ID NO:20), Lys-Xaa-Xaa-Lys-Lys-Lys (SEQ ID NO:21), Lys-Xaa-Lys-Xaa-Lys-Lys (SEQ ID NO:22), Lys-Xaa-Lys-Lys-Xaa-Lys (SEQ ID NO:23), Lys-Xaa-Lys-Lys-Lys-Xaa (SEQ ID NO:24), Lys-Lys-Xaa-Xaa-Lys-Lys (SEQ ID NO:25), Lys-Lys-Xaa-Lys-Xaa-Lys (SEQ ID NO:26), Lys-Lys-Xaa-Lys-Lys-Xaa (SEQ ID NO:27), Lys-Lys-Lys-Xaa-Xaa-Lys (SEQ ID NO:28), Lys-Lys-Lys-Xaa-Lys-Xaa (SEQ ID NO:29), Lys-Lys-Lys-Lys-Xaa-Xaa, Xaa-Xaa-Xaa-Lys-Lys-Lys, Xaa-Xaa-Lys-Xaa-Lys-Lys, Xaa-Xaa-Lys-Lys-Xaa-Lys, Xaa-Xaa-Lys-Lys-Lys-Xaa, Xaa-Lys-Xaa-Xaa-Lys-Lys, Xaa-Lys-Xaa-Lys-Xaa-Lys, Xaa-Lys-Xaa-Lys-Lys-Xaa, Xaa-Lys-Lys-Xaa-Xaa-Lys, Xaa-Lys-Lys-Xaa-Lys-Xaa, Xaa-Lys-Lys-Lys-Xaa-Xaa, Lys-Lys-Lys-Xaa-Xaa-Xaa, Lys-Lys-Xaa-Lys-Xaa-Xaa, Lys-Lys-Xaa-Xaa-Lys-Xaa, Lys-Lys-Xaa-Xaa-Xaa-Lys, Lys-Xaa-Lys-Lys-Xaa-Xaa, Lys-Xaa-Lys-Xaa-Lys-Xaa, Lys-Xaa-Lys-Xaa-Xaa-Lys, Lys-Xaa-Xaa-Lys-Lys-Xaa, Lys-Xaa-Xaa-Lys-Xaa-Lys, Lys-Xaa-Xaa-Xaa-Lys-Lys, Lys-Lys-Xaa-Xaa-Xaa-Xaa, Lys-Xaa-Lys-Xaa-Xaa-Xaa, Lys-Xaa-Xaa-Lys-Xaa-Xaa-Lys, Lys-Xaa-Xaa-Xaa-Lys-Xaa-Lys, Lys-Xaa-Xaa-Xaa-Xaa-Lys-Lys, Xaa-Lys-Lys-Xaa-Xaa-Xaa, Xaa-Lys-Xaa-Lys-Xaa-Xaa, Xaa-Lys-Xaa-Xaa-Lys-Xaa, Xaa-Lys-Xaa-Xaa-Xaa-Lys, Xaa-Xaa-Lys-Lys-Xaa-Xaa, Xaa-Xaa-Lys-Xaa-Lys-Xaa, Xaa-Xaa-Lys-Xaa-Xaa-Lys, Xaa-Xaa-Xaa-Lys-Lys-Xaa, Xaa-Xaa-Xaa-Lys-Xaa-Lys, Xaa-Xaa-Xaa-Xaa-Lys-Lys, Lys-Xaa-Xaa-Xaa-Xaa-Xaa, Xaa-Lys-Xaa-Xaa-Xaa-Xaa, Xaa-Xaa-Lys-Xaa-Xaa-Xaa, Xaa-Xaa-Xaa-Lys-Xaa-Xaa, Xaa-Xaa-Xaa-Xaa-Lys-Xaa, Xaa-Xaa-Xaa-Xaa-Xaa-Lys, Xaa-Xaa-Xaa-Xaa-Xaa-Xaa, Lys-Lys-Lys-Lys-Lys-Lys-Lys (SEQ ID NO:30), Xaa-Lys-Lys-Lys-Lys-Lys-Lys (SEQ ID NO:31), Lys-Xaa-Lys-Lys-Lys-Lys-Lys (SEQ ID NO:32), Lys-Lys-Xaa-Lys-Lys-Lys-Lys (SEQ ID NO:33), Lys-Lys-Lys-Xaa-Lys-Lys-Lys (SEQ ID NO:34), Lys-Lys-Lys-Lys-Xaa-Lys-Lys (SEQ ID NO:35), Lys-Lys-Lys-Lys-Lys-Xaa-Lys (SEQ ID NO:36), Lys-Lys-Lys-Lys-Lys-Lys-Xaa (SEQ ID NO:37), Xaa-Xaa-Lys-Lys-Lys-Lys-Lys (SEQ ID NO:38), Xaa-Lys-Xaa-Lys-Lys-Lys-Lys (SEQ ID NO:39), Xaa-Lys-Lys-Xaa-Lys-Lys-Lys (SEQ ID NO:40), Xaa-Lys-Lys-Lys-Xaa-Lys-Lys (SEQ ID NO:41), Xaa-Lys-Lys-Lys-Lys-Xaa-Lys (SEQ ID NO:42), Lys-Xaa-Xaa-Lys-Lys-Lys-Lys (SEQ ID NO:43), Lys-Xaa-Lys-Xaa-Lys-Lys-Lys (SEQ ID NO:44), Lys-Xaa-Lys-Lys-Xaa-Lys-Lys (SEQ ID NO:45), Lys-Xaa-Lys-Lys-Lys-Xaa-Lys (SEQ ID NO:46), Lys-Lys-Xaa-Xaa-Lys-Lys-Lys (SEQ ID NO:47), Lys-Lys-Xaa-Lys-Xaa-Lys-Lys (SEQ ID NO:48), Lys-Lys-Xaa-Lys-Lys-Xaa-Lys (SEQ ID NO:49), Lys-Lys-Lys-Xaa-Xaa-Lys-Lys (SEQ ID NO:50), Lys-Lys-Lys-Xaa-Lys-Xaa-Lys (SEQ ID NO:51), Lys-Lys-Lys-Lys-Xaa-Xaa-Lys (SEQ ID NO:52), Xaa-Xaa-Xaa-Lys-Lys-Lys-Lys (SEQ ID NO:53), Xaa-Xaa-Lys-Xaa-Lys-Lys-Lys (SEQ ID NO:54), Xaa-Xaa-Lys-Lys-Xaa-Lys-Lys (SEQ ID NO:55), Xaa-Xaa-Lys-Lys-Lys-Xaa-Lys (SEQ ID NO:56), Xaa-Lys-Xaa-Xaa-Lys-Lys-Lys (SEQ ID NO:57), Xaa-Lys-Xaa-Lys-Xaa-Lys-Lys (SEQ ID NO:58), Xaa-Lys-Xaa-Lys-Lys-Xaa-Lys (SEQ ID NO:59), Xaa-Lys-Lys-Xaa-Xaa-Lys-Lys (SEQ ID NO:60), Xaa-Lys-Lys-Xaa-Lys-Xaa-Lys (SEQ ID NO:61), Xaa-Lys-Lys-Lys-Xaa-Lys-Xaa (SEQ ID NO:62), Xaa-Lys-Lys-Xaa-Lys-Lys-Xaa (SEQ ID NO:63), Xaa-Lys-Xaa-Lys-Lys-Lys-Xaa (SEQ ID NO:64), Xaa-Lys-Lys-Lys-Xaa-Xaa-Lys (SEQ ID NO:65), Lys-Xaa-Lys-Lys-Lys-Xaa-Xaa (SEQ ID NO:66), Xaa-Lys-Lys-Lys-Lys-Xaa-Xaa (SEQ ID NO:67), Xaa-Lys-Lys-Lys-Xaa-Lys-Xaa (SEQ ID NO:68), Xaa-Lys-Lys-Lys-Xaa-Xaa-Lys (SEQ ID NO:69), Lys-Lys-Lys-Lys-Xaa-Xaa-Xaa (SEQ ID NO:70), Lys-Lys-Lys-Xaa-Xaa-Xaa-Lys (SEQ ID NO:71), Lys-Lys-Lys-Xaa-Lys-Xaa-Xaa (SEQ ID NO:72), Lys-Lys-Xaa-Lys-Lys-Xaa-Xaa (SEQ ID NO:73), Lys-Lys-Xaa-Xaa-Lys-Xaa-Lys (SEQ ID NO:74), Lys-Lys-Xaa-Xaa-Xaa-Lys-Lys (SEQ ID NO:75), Lys-Lys-Xaa-Lys-Lys-Xaa-Xaa (SEQ ID NO:76), Lys-Xaa-Lys-Lys-Xaa-Xaa-Lys (SEQ ID NO:77), Lys-Xaa-Lys-Xaa-Lys-Xaa-Lys (SEQ ID NO:78), Lys-Xaa-Lys-Xaa-Xaa-Lys-Lys (SEQ ID NO:79), Lys-Xaa-Xaa-Lys-Lys-Xaa-Lys (SEQ ID NO:80), Lys-Xaa-Xaa-Lys-Xaa-Lys-Lys (SEQ ID NO:81), Lys-Xaa-Xaa-Xaa-Lys-Lys-Lys (SEQ ID NO:82), Lys-Lys-Xaa-Xaa-Xaa-Xaa-Lys, Lys-Xaa-Lys-Xaa-Xaa-Xaa-Lys, Lys-Xaa-Xaa-Lys-Xaa-Xaa-Lys, Lys-Xaa-Xaa-Xaa-Lys-Xaa-Lys, Lys-Xaa-Xaa-Xaa-Xaa-Lys-Lys, Xaa-Lys-Lys-Xaa-Xaa-Xaa-Lys, Xaa-Lys-Xaa-Lys-Xaa-Xaa-Lys, Xaa-Lys-Xaa-Xaa-Lys-Xaa-Lys, Xaa-Lys-Xaa-Xaa-Xaa-Lys-Lys, Xaa-Xaa-Lys-Lys-Xaa-Xaa-Lys, Xaa-Xaa-Lys-Xaa-Lys-Xaa-Lys, Xaa-Xaa-Lys-Xaa-Xaa-Lys-Lys, Xaa-Xaa-Xaa-Lys-Lys-Xaa-Lys, Xaa-Xaa-Xaa-Lys-Xaa-Lys-Lys, Xaa-Xaa-Xaa-Xaa-Lys-Lys-Lys, Lys-Xaa-Xaa-Xaa-Xaa-Xaa-Lys, Xaa-Lys-Xaa-Xaa-Xaa-Xaa-Lys, Xaa-Xaa-Lys-Xaa-Xaa-Xaa-Lys, Xaa-Xaa-Xaa-Lys-Xaa-Xaa-Lys, Xaa-Xaa-Xaa-Xaa-Lys-Xaa-Lys, Xaa-Xaa-Xaa-Xaa-Xaa-Lys-Lys, Xaa-Xaa-Xaa-Xaa-Xaa-Xaa-Lys, Lys-Xaa-Xaa-Xaa-Xaa-Xaa-Xaa, Xaa-Xaa-Xaa-Xaa-Xaa-Lys-Xaa, Xaa-Lys-Xaa-Xaa-Xaa-Xaa-Xaa, Xaa-Xaa-Lys-Xaa-Xaa-Xaa, Xaa-Xaa-Xaa-Xaa-Lys-Xaa, Xaa-Xaa-Xaa-Xaa-Xaa-Xaa-Xaa, wherein each Xaa is independently selected from the group consisting of Ala, Leu, Ser, Thr, Tyr, Asn, Gln, Asp, Glu, Arg, His, Met, Orn, and amino acids of the formula I as defined herein, e.g., Dbu or Dpr.

As indicated above, the amino acid residues of Z may of course all be different or all be identical. However, in interesting embodiments of the present invention, the amino acid residues in Z are selected from two or three different amino acids, or are identical amino acids. Examples of suitable peptide sequences, wherein the amino acid residues in Z are identical are e.g., (Lys)_{n,} wherein n is an integer in the range from 4 to 7, e.g., Lys₄ (SEQ ID NO:1), Lys₅ (SEQ ID NO:2), Lys₆ (SEQ ID NO:8), Lys₇ (SEQ ID NO:30). Preferred is (Lys)₆ bound via a peptide bond to the C-terminal of X.

Examples of suitable peptide sequences, wherein the amino acid residues in Z are selected from about two different amino acids are e.g., (Lys-Xaa)ₘ or (Xaa-Lys)_{m,} wherein m is an integer in the range from about 2 to 7, preferably in the range from 2 to 5, such as in the range from 2 to 4, e.g., 3, and Xaa is independently selected from the group consisting of Ser, Thr, Tyr, Asn, Gin, Asp, Glu, Arg, His, Orn, 2,4-diaminobutanoic acid, 2,3-diaminopropanoic acid and Met. More preferably such peptide sequences are e.g., (Lys-Xaa)₃ or (Xaa-Lys)_{3,} wherein Xaa is as defined above, such as (Lys-Glu)₃ (SEQ ID NO:83) or (Glu-Lys)₃ (SEQ ID NO:84). Other examples of suitable peptide sequences, wherein the amino acid residues in Z are selected from about two amino acid residues are e.g., Lysₚ-Xaa _{q} or Xaaₚ-Lys_{q,} wherein p and q are integers in the range from 1 to 14, with the proviso that p+q is in the range from 4 to 15, preferably in the range from 4 to 10, such as in the range from 4 to 8, e.g., in the range from 4 to 6, e.g., 4, 5 or 6, and Xaa is independently selected from the group consisting of Ser, Thr, Tyr, Asn, Gln, Asp, Glu, Arg, His and Met. More preferably such peptide sequences are e.g., Lys₃-Xaa₃ or Xaa₃-Lys_{3,} wherein Xaa is as defined above, such as Lys₃-Glu₃ (SEQ ID NO:85) or Glu₃-Lys₃ (SEQ ID NO:86). More preferred Z sequences consists of a sequence of amino acid residues selected from Asn and Gln together with 4-7 amino acid residues selected from Glu and Asp, such as Asn-(Glu)_{5,} Asn-(Glu)_{6,} Gln-(Glu)_{5,} Asn-(Asp)_{5,} and Gin-(Asp)_{5,} which is the N-terminal part of the peptide conjugate of the invention.

Examples of suitable peptide sequences, wherein the amino acid residues in Z are selected from three different amino acids are e.g., Xaa¹-(Lys)ₓ-(Xaa²)_{y,} Xaa¹-(Xaa²)ₓ-(Lys)_{y,} (Lys)ₓ-(Xaa²)_{y}-Xaa¹, (Xaa¹ )ₓ-(Lys)_{y}-Xaa², (Lys)ₓ-Xaa¹-(Xaa²)_{y,} (Xaa¹)ₓ-Xaa²-(Lys)_{y,} Xaa¹-Lys-Xaa²-Lys, Xaa¹-Lys-Xaa²-Lys-Xaa², Xaa¹-Lys-Xaa²-Lys-Xaa² -Lys, Xaa¹-Xaa²-Lys-Xaa², Xaa¹ -Xaa²-Lys-Xaa²-Lys, Xaa¹-Xaa¹-Lys-Xaa²-Lys-Xaa², Lys-Xaa²-Lys-Xaa¹, Lys-Xaa²-Lys-Xaa²-Xaa¹, Lys-Xaa²-Lys-Xaa²-Lys-Xaa¹, Xaa²-Lys-Xaa²-Xaa¹, Xaa²-Lys-Xaa²-Lys-Xaa¹, Xaa²-Lys-Xaa¹ -Lys-Xaa²-Xaa¹, etc., wherein x and y are integers in the range from about 1 to 5 with the proviso that x+y is at the most 6, and Xaa¹ and Xaa² is independently selected from about the group consisting of Ala, Leu, Ser, Thr, Tyr, Asn, Gln, Asp, Glu, Arg, His, Met, Orn, 2,3-diaminopropanoic acid, 2,4-diaminobutanoic acid and amino acids of the formula I as defined herein.

A most preferred embodiment of the invention is directed to a novel peptide conjugate comprising a peptide X being an agonist of exendin-4 activity selected from the group consisting of des Pro³⁶-exendin-4(1-39)-NH₂ (SEQ ID NO:101),
des Pro³⁶-exendin-4(1-40)-NH_{2,}
des Pro³⁶-des Pro³⁷ -exendin4(1-39)-NH_{2,}
des Pro³⁶-des Pro³⁷-des Pro³⁸-exendin-4(1-39)-NH_{2,}
des Pro³⁶-des Pro³⁷-des Pro³⁸-exendin-4(1-40)-NH_{2,}
des Ala³⁵-exendin-4(1-39)-NH₂ (SEQ IDNO:105),
des Gly³⁴-exendin-4(1-39)-NH₂ (SEQ ID NO:106),
des Gly³⁴ -(Lys⁴⁰ (palmitoyl))exendin-4(1-39)-NH₂ (SEQ ID NO:108),
des Ala³⁵-(Lys⁴⁰ (palmitoyl))exendin-4(1-39)-NH₂ (SEQ ID NO:109),
des Pro³⁶ Lys⁴⁰ (palmitoyl))exendin-4(1-39)-NH₂ (SEQ ID NO:110).

It should be understood that the peptide conjugates of the invention might also be in the preferred amide (NH₂) or in the free acid (OH) form or in the form of a salt thereof. Exemplary peptide conjugates of the invention are
des Ser³⁹-exendin-4(1-39)-(Lys)₆-NH₂ (SEQ ID NO:91),
exendin-4(1-39)-Lys₆-NH₂ (SEQ ID NO:92),
des Pro³⁶-exendin-4(1-39)-Lys₆-NH₂ (SEQ ID NO:93),
des Ala³⁵-exendin-4(1-39)-Lys₆-NH₂ (SEQ ID NO:94),
des Gly³⁴-exendin-4(1-39)-Lys₆-NH₂ (SEQ ID NO:95),
des Ser³⁹-(Lys⁴⁰ (palmitoyl))exendin-4(1-39)-Lys₇-NH₂ (SEQ ID NO:96),
des Gly³⁴-(Lys⁴⁰ (palmitoyl))exendin-4(1-39)-Lys₇-NH₂ (SEQ ID NO:97),
des Ala³⁵-(Lys⁴⁰ (palmitoyl))exendin-4(1-39)-Lys₇-NH₂ (SEQ ID NO:98),
des pro³⁶-(Lys⁴⁰ (palmitoyl))exendin-4(1-39)-Lys₇-NH₂ (SEQ ID NO:99),
Lys⁴⁰ (palmitoyl)exendin-4(1-39)-Lys₇-NH₂ (SEQ ID NO:100),
des Pro³⁶, Pro³⁷-exendin-4(1-39)-Lys₆-NH_{2,}
Asn(Glu)₅-des Pro³⁶, Pro³⁷, Pro³⁸-exendin-4(1-39)-Lys₆ NH_{2,}
des Pro³⁶, Pro³⁷, Pro³⁸-exendin-4(1-39)-Lys₆-NH₂
and the free acid thereof and a pharmaceutically acceptable salt thereof.
Among the Preferred conjugates are
des Pro³⁶ -exendin-4(1-39)-Lys₆-NH₂ (SEQ ID NO:93),
des Pro³⁶, Pro³⁷, Pro³⁸-exendin-4(1-39)-Lys₆-NH₂, and
their salts as defined herein.

In a most specific embodiment, the conjugates are selected from the group consisting of des Ser³⁹-exendin-4(1-39)-Lys₆-NH_{2,} exendin-4(1-39)-Lys₆-NH_{2,} des Pro³⁶-exendin-4(1-39)-Lys₆-NH_{2,} des Ala³⁵-exendin-4(1-39)-Lys₆-NH_{2,} des Gly³⁴-exendin-4(1-39)-Lys₆-NH_{2,} des Ser³⁹-(Lys⁴⁰ (palmitoyl))exendin-4(1-39)-Lys₇-NH_{2,} des Gly³⁴ -(Lys⁴⁰ (palmitoyl))exendin-4(1-39)-Lys₇-NH_{2,} des Ala³⁵-(Lys⁴⁰ (palmitoyl))exendin-4(1-39)-Lys₇-NH_{2,} des Pro³⁶(Lys⁴⁰ (palmitoyl))exendin-4(1-39)-Lys₇-NH₂ and Lys⁴⁰ (palmitoyl)exendin-4(1-39)-Lys₇-NH₂.

The provision of the peptide conjugates of the present invention enables blood glucose lowering exendin peptide conjugates and their active analogues to be administered orally. The herein preferred terminal peptide fragments Z are chosen so as to induce an alpha-helical structure to the peptide X without significantly affecting the desired activity of X. Said helical structure stabilises the peptide chain, e.g. againsts degradation, as evidenced by the increased half life of from 2 to 3 times of the conjugated peptide compared to the unconjugated peptide, cf. table 5 below. The peptide sequence Z is the part of the peptide conjugate responsible for introducing of a certain structure into the molecule so that the minimum effective dose is lowered at least five fold. Preferably the minimum effective dose is lowered at least ten fold, more preferably 25 fold, even more preferably 40 fold, and most preferably 50 fold. Therefore, the present invention also relates to the use of a peptide sequence (Z) as defined above for the preparation of a said peptide conjugate as defined above.

Thus, the invention also relates to a novel peptide conjugate comprising a peptide X as defined herein and wherein X reduces the blood glucose level in a mammal where the ratio between the minimum effective oral dose of said peptide conjugate and the minimum effective oral dose of the peptide X is at least 1:5.

Specifically, the invention provides the medical use of the exendin-4 conjugates, specifically in treatment of diabetes type 1 or type 2, obesity, eating disorders, hyperglycemia, metabolic disorders, gastric disease and insulin resistance syndrome.

The present invention also relates to methods for the preparation of said peptide conjugate, by means of recombinant DNA technology comprising the steps of (a) introducing a nucleic acid sequence encoding said conjugate into a host cell and (b) culturing said host cell and (c) isolating said conjugate from the culture or (a) culturing a recombinant host cell comprising a nucleic acid sequence encoding said conjugate under conditions permitting the production of said conjugate and (b) isolating said conjugate from the culture.

The method also relates to methods for the preparation of said peptide conjugate in which peptide X is obtained via recombinant DNA methods by isolating said peptide. X is then conjugated to Z which is attached to a solid support or has been prepared by solid phase synthetic methods.
Furthermore, the invention relates to the preparation of the peptide conjugate of the present invention by peptide synthetic methods. Furthermore, the invention relates to the preparation of the peptide conjugate of the present invention by peptide synthetic methods.

The conjugates of the invention comprising an N-terminal sequence of from 33 to 39, preferably from 36 to 38, amino acid residues having a substantial homology to the native exendin-4 N-terminal sequence thought to be essential for receptor binding (insulinotropic activity) and a C-terminal sequence Z possess as a further advantage improved stability compared to native exendins and C-terminally truncated forms of exendin.

### Compositions

The invention also concerns a composition comprising the exendin-4 peptide conjugate of the present invention in combination with a physiologically acceptable carrier. Such compositions may be in a form adapted to oral, parenteral (including subcutaneous (s.c.), intravenous (i.v.), intramuscular (i.m.), epidural, direct brain and intraperitoneal (i.p.)), rectal, intratracheal, intranasal, dermal, vaginal, buccal, ocularly, or pulmonary administration, preferably in a form adapted to subcutaneous or oral administration, and such compositions may be prepared in a manner well-known to the person skilled in the art, e.g., as generally described in "Remington's Pharmaceutical Sciences", 17. Ed. Alfonso R. Gennaro (Ed.), Mark Publishing Company, Easton, PA, U.S.A., 1985 and more recent editions and in the monographs in the "Drugs and the Pharmaceutical Sciences" series, Marcel Dekker. The compositions may appear in conventional forms, for example, capsules, tablets, aerosols, topical application forms, liquid or semiliquid forms, such as solutions, suspensions, dispersions, emulsions, micelles or liposomes. Preferred are liquid compositions suitable for s.c. administration. In a preferred embodiment, the compositions of the present invention are administered subcutaneously. In an alternative preferred embodiment, the compositions of the present invention are administered orally, and in such cases one preferred administration form is a tablet or capsule.

The pharmaceutical carrier or diluent employed may be a conventional solid or liquid carrier. Examples of solid carriers are lactose, terra alba, sucrose, cyclodextrin, talc, gelatin, agar, pectin, acacia, magnesium stearate, stearic acid ro lower alkyl ethers of cellulose. Examples of liquid carriers are syrup, peanut oil, olive oil, phospholipids, sterols, fatty acids, fatty acid amines, polyoxyethylene, isotonic buffer solutions and water. Similarly, the carrier or diluent may include any sustained release material known in the art, such as glyceryl monostearate or glyceryl distearate, alone or mixed with a wax. If a solid carrier is used for oral administration, the preparation may be tabletted, placed in a hard gelatin capsule in powder or pellet form or it can be in the form of a troche or lozenge. The amount of solid carrier will vary widely but will usually be from about about 25 mg to about 1 g.

A typical tablet which may be prepared by conventional tabletting techniques may contain:
- Core: active compound (as free compound of the invention or salt thereof) 100 mg; colloidal silicon dioxide (Aerosil) 1.5 mg; cellulose, microcryst. (Avicel) 70 mg; modified cellulose gum (Ac-Di-Sol) 7.5 mg; magnesium stearate.
- Coating: HPMC approx. 9 mg; *Mywacett 9-40T approx. 0.9 mg; *acylated monoglyceride used as plasticizer for film coating.

If a liquid carrier is used, the preparation may be in the form of a syrup, emulsion, soft gelatin capsule or sterile injectable liquid such as an aqueous or non-aqueous liquid suspension or solution.

For nasal administration, the preparation may contain a compound of the present invention, preferably a conjugate, dissolved or suspended in a liquid carrier, in particular, an aqueous carrier, for aerosol application. The carrier may contain additives such as solubilizing agents, e.g., propylene glycol, surfactants such as bile acid salts or polyoxyethylene higher alcohol ethers, absorption enhancers such as lecithin (phosphatidylcholine) or cyclodextrin, or preservatives such as parabines.

The composition may also be in a form suited for local or systemic injection or infusion and may, as such, be formulated with sterile water or an isotonic saline or glucose solution. The compositions may be sterilized by conventional sterilization techniques which are well known in the art. The resulting aqueous solutions may be packaged for use or filtered under aseptic conditions and lyophilized, the lyophilized preparation being combined with the sterile aqueous solution prior to administration. Preferably, the formulation to be used for intravenous, subcutaneous and oral dosing will be a solution of the active compound in buffer.The preparation may be produced immediately before use from active drug substance and sterile buffer solution. One preferred method of sterilization may be by sterile filtration of a solution made immediately prior to use. The composition may contain pharmaceutically acceptable auxiliary substances as required to approximate physiological conditions, such as buffering agents, tonicity adjusting agents and the like, for instance sodium acetate, sodium lactate, sodium chloride, potassium chloride, calcium chloride, etc.

The compounds of the invention possess valuable pharmacological properties, e.g. stability towards proteolytic enzymes. In vitro stability studies with the present peptides and peptide conjugates in the presence of selected proteolytic enzymes show increased half lives of the novel peptides compared to prior art peptides. Thus, the compounds of the invention exhibit considerably extended duration of action in vivo compared to GLP-1 and other GLP-1 agonists. Furthermore, the compounds of the invention stimulate cAMP formation. This effect may be demonstrated in a cAMP assay, e.g. as described in WO 98/08871.

The peptide compounds of the present invention are agonists exendin-4 activity and improves blood glucose tolerance in diabetic mammals as determined by assays known in the art for a particular peptide. Examples of such an assay are described herein. Thus, the invention also concerns the exendin variants and peptide conjugates as defined above for use in therapy, and the use of the peptide conjugates as defined above for the manufacture of a pharmaceutical composition for use in therapy, e.g., in the treatment of diabetes type 1 or type 2, obesity, eating disorders and insulin resistance syndrome.

In specific embodiments, the exendin variants and peptide conjugates of the invention may be used to stimulate insulin release, lower blood glucose level, reduce gastric motility, delay gastric emptying, inhibit food uptake, e.g. by suppression of appetite, or lower the plasma lipid level in a vertebrate or a mammal. The novel compounds of the invention may also be used generally in the treatment of diabetes mellitus associated with a risk for hyperglycemia, i.e. where insulin sensitivity is decreased with stress, myocardia infection, stroke and infections, or in cases of insulin resistance during pregnancy. The novel compounds may also be used in the treatment of other types of diabetes, such as cases where diabetes may be secondary to other endocrine diseases such as acromegaly, Cushing's syndrome, pheochromocytoma, glucagonoma, somatostatinoma, primary aldosteronism, or secondary to adminstration of certain hormones causing hyperglycemia, or secondary to certain drugs (antihypertensive drugs, thiazide diuretics, preparations containing estrogen, psychoactive drugs, sympathomimetic agents. Furthermore, the novel compounds of the invention may be used generally in the treatment of diseases and conditions associated with a risk for hypoglycemia, i.e. where endogenous glucose production is decreased, as following alcohol ingestion, or in cases where the sensitivity to insulin is increased in patients with hypopituitarism or primary adrenocortical insufficiency, or where insulin clearance is devreased as with progressive renal insufficieny.

Other specific therapeutic uses are described in WO 99/40788 (relating to the inotropic and diuretic effects of exendin and GLP-1) WO 98/39022 (relating to a method of sedating a mammalian subject having increased activation of the central or peripheral nervous system comprising administering exendin or GLP-1 or an agonist of exendin or GLP-1 to the subject to produce a sedative or anxiolytic effect on the subject), WO 93/18786 (relating to the treatment of diabetes using GLP-1(7-37) or GLP-1(7-36)amide in a regimen which additionally comprises treatment with an oral hypoglycaemic agent, such as sulfonylurea, producing a strong synergistic effect), WO 98/19698 (relating to the use of GLP-1 analogs for the regulation of obesity), WO 98/08531 (relating to the use of GLP-1 or analogs in a method of reducing mortality and morbidity after myocardial infarction), WO 98/08873 (relating to the use of GLP-1 or analogs in a method of attenuating post-surgical catabolic changes and hormonal responses to stress). Besides, the compounds of the invention are suitable in a combination therapy with other antidiabetic agents, such as insulin, metformin, sulfonyl ureas and thiazolidinediones, or in combination therapy with other antiobesity agents, such as leptin, dexphenfluramine, amphetamin etc..

### Definitions

A "peptide" as used herein is any compound produced by amide formation between a carboxyl group of one amino acid and an amino group of another. The amide bonds in peptides may be called peptide bonds. The word peptide usually applies to compounds whose amide bonds are formed between C-1 of one amino acid and N-2 of another (sometimes called eupeptide bonds), but it includes compounds with residues linked by other amide bonds (sometimes called isopeptide bonds). Peptides with fewer than about 10-20 residues may also be called oligopeptides; those with more, polypeptides. Polypeptides of specific sequence of more than about 50 residues are usually known as proteins. A "natural polypeptide sequence" as used herein refers to a polypeptide sequence consisting of natural L-amino acid residues and which is capable of being expressed by a recombinant host cell. The X compounds herein are all peptide sequences of 40 amino acid residues or less.

"Agonist" refers to an endogenous substance or a drug that can interact with a receptor and initiate a physiological or a pharmacological response characteristic of that receptor (contraction, relaxation, secretion, enzyme activation, etc.).

"Antagonist" refers to a drug or a compound that opposes the physiological effects of another. At the receptor level, it is a chemical entity that opposes the receptor-associated responses normally induced by another bioactive agent.

"Partial agonist" refers to an agonist which is unable to induce maximal activation of a receptor population, regardless of the amount of drug applied. A "partial agonist" may be termed "agonist with intermediate intrinsic efficacy" in a given tissue. Moreover, a partial agonist may antagonize the effect of a full agonist that acts on the same receptor.

"Receptor" refers to a molecule or a polymeric structure in or on a cell that specifically recognizes and binds a compound acting as a molecular messenger (neurotransmitter, hormone, lymphokine, lectin, drug, etc.).

"Exendin-4" as used herein refers to exendin-4(1-39) the amino acid sequence of which is disclosed in US patent No. 5,424,286, SEQ ID NO:2, and exendin-4(1-40) as disclosed by Chen & Drucker in The Journal of Biological Chemistry, Vol. 272, No. 7, pp.4108-15 which differs only in having glycine in position 40 as C-terminal amino acid residue. The homology of the parent exendin is determined as the degree of identity between two protein sequences indicating a derivation of the first sequence from the second. The homology may suitably be determined by means of computer programs known in the art such as GAP provided in the GCG program package (Program Manual for the Wisconsin Package, Version 8, August 1994, Genetics Computer Group, 575 Science Drive, Madison, Wisconsin, USA 53711) (Needleman, S.B. and Wunsch, C.D., (1970), J. Mol. Biol. 48:443-453). The following settings for polypeptide sequence comparison may be used: GAP creation penalty of 3.0 and GAP extension penalty of 0.1.

"Salts" include pharmaceutically acceptable salts, such as acid addition salts and basic salts. Examples of acid addition salts are hydrochloride salts, sodium salts, hydrobromide salts, etc. Examples of basic salts are salts where the cation is selected from alkali metals, such as sodium and potassium, alkaline earth metals, such as calcium, and ammonium ions ⁺N(R³)₃(R⁴), where R³ and R⁴ independently designates optionally substituted C₁₋₆-alkyl, optionally substituted C₂₋₆-alkenyl, optionally substituted aryl, or optionally substituted heteroaryl. Other examples of pharmaceutically acceptable salts are; e.g., those described in "Remington's Pharmaceutical Sciences" 17. Ed. Alfonso R. Gennaro (Ed.), Mark Publishing Company, Easton, PA, U.S.A., 1985 and more recent editions, and in Encyclopedia of Pharmaceutical Technology.

### Preparation of Variants and Conjugates

The exendin variants and the peptide conjugates of the invention may be prepared by methods known per se in the art. Thus, the variants and the peptide sequences X and Z may be prepared by standard peptide-preparation techniques such as solution synthesis or Merrifield-type solid phase synthesis. It is believed that the Boc (tert.butyloxycarbonyl) as well as the Fmoc (9-fluorenylmethyloxycarbonyl) strategies are applicable.

In one possible synthesis strategy, the peptide conjugates of the invention may be prepared by solid phase synthesis by first constructing the peptide sequence Z using well-known standard protection, coupling and deprotection procedures, thereafter sequentially coupling the exendin on Z in a manner similar to the construction of Z, and finally cleaving off the entire peptide conjugate from the carrier. This strategy yields a peptide conjugate, wherein the peptide sequence Z is covalently bound to the peptide X at the C-terminal carbonyl function of X. If the desired peptide conjugate, however, is a peptide conjugate, wherein two stabilising sequences Z are covalently and independently bound to both the C- and the N-terminal of the peptide X, the above strategy is also applicable but, as will be understood by the person skilled in the art, before cleaving the off the C-terminal bound peptide conjugate from the solid support, it is necessary to sequentially couple the second peptide sequence Z to the N-terminal of X in a manner similar to the procedure described above. This strategy may also be used to attach Z to the carbonyl function on the side chain of Glu or Asp. A possible strategy for the preparation of peptide conjugates, wherein the peptide sequence Z is covalently bound to the N-terminal nitrogen atom or covalently bound to the nitrogen atom on the side chain of Lys, Arg or His of X is analogous with the method described above, i.e. said peptide conjugates may be prepared by solid phase synthesis by first constructing the peptide sequence X using well-known standard protection, coupling and deprotection procedures, thereafter sequentially coupling the peptide sequence Z on X in a manner similar to the construction of X, and finally cleaving off the entire peptide conjugate from the carrier. Another possible strategy is to prepare one or both of the two sequences X and Z (or parts thereof) separately by solution synthesis, solid phase synthesis, recombinant techniques, or enzymatic synthesis, followed by coupling of the two sequences by well-known segment condensation procedures, either in solution or using solid phase techniques or a combination thereof. In one embodiment, X may be prepared by recombinant DNA methods and Z may be prepared by solid phase synthesis. The conjugation of X and Z may be carried out by using chemical ligation. This technique allows for the assembling of totally unprotected peptide segments in a highly specific manner (Liu et al., 1996, J. Am. Chem. Soc. 118:307-312 and Dawson et al., 1996, 226:776). The conjugation can also be performed by protease-catalysed peptide bond formation, which offers a highly specific technique to combine totally unprotected peptide segments via a peptide bond (W. Kullmann, 1987, Enzymatic Peptide Synthesis, CRC Press, Boca Raton, Florida, pp. 41-59).

Side chain derivaiization of Lys, Arg, His, Trp, Ser, Thr, Cys, Tyr, Asp and Glu with the peptide sequence, Z, can be carried out by traditional convergent peptide synthesis using suitable orthogonal protecting schemes as known in the art, or by using the equally well known general solid phase method with suitable orthogonal removable chain protection.

Furthermore, it is envisaged that a combination of the above-mentioned strategies may be especially applicable where a modified peptide sequence, e.g., from a peptide X comprising isosteric bonds such as reduced peptide bonds, is to be coupled to a peptide sequence Z. In this case, it may be advantageous to prepare the immobilised fragment of Z by successive coupling of amino acids, and then couple a complete peptide sequence X (prepared in solution or fully or partially using solid phase techniques or by means of recombinant techniques) to the fragment.

Examples of suitable solid support materials (SSM) are e.g., functionalised resins such as polystyrene, polyacrylamide, polydimethylacrylamide, polyethyleneglycol, cellulose, polyethylene, polyethyleneglycol grafted on polystyrene, latex, dynabeads, etc. It should be understood that it may be necessary or desirable that the C-terminal amino acid of the peptide sequence Z or the C-terminal amino acid of the peptide X is attached to the solid support material by means of a common linker such as 2,4-dimethoxy-4'-hydroxy-benzophenone, 4-(4-hydroxy-methyl-3-methoxyphenoxy)-butyric acid, 4-hydroxy-methylbenzoic acid, 4-hydroxymethyl-phenoxyacetic acid, 3-(4-hydroxymethylphenoxy)propionic acid, and p-[(R,S)-a[1-(9H-fluoren-9-yl)methoxyformamido]-2,4-dimethoxybenzyl]-phenoxy-acetic acid.

The variants and the peptide conjugates of the invention may be cleaved from the solid support material by means of an acid such as trifluoracetic acid, trifluoromethanesulfonic acid, hydrogen bromide, hydrogen chloride, hydrogen fluoride, etc. optionally in combination with one or more "scavengers" suitable for the purpose, e.g., ethanedithiol, triisopropylsilane, phenol, thioanisole, etc., or the peptide conjugate of the invention may be cleaved from the solid support by means of a base such as ammonia, hydrazine, an alkoxide, such as sodium ethoxide, an hydroxide, such as sodium hydroxide, etc.

Thus, the peptide conjugates of the present invention can be prepared in a method for the preparation of a peptide conjugate of formula I (X-Z), which comprises the steps of:
a) coupling an amino acid or dipeptide having suitable protecting groups, including an N-α-protecting group, in the activated form to an immobilised peptide sequence H-Z-SSM, thereby forming an immobilised N-α-protected peptide fragment,
b) removing said N-α-protecting group, thereby forming an immobilised protected peptide fragment having an unprotected N-terminal,
c) coupling an additional amino acid or dipeptide having suitable protecting groups including an N-α-protecting group in the carboxyl activated form to the N-terminal of the immobilised peptide fragment, and repeating the removal/coupling step procedure in step b) and c) until the desired peptide sequence X is obtained, and then
d) cleaving off the peptide conjugate from the solid support material.

Furthermore, a method for the preparation of a peptide conjugate of formula III (Z-X-Z), comprises the steps of:
a) coupling an amino acid or dipeptide having suitable protecting groups, including an N-α-protecting group, in the carboxyl activated form to an immobilised peptide sequence H-Z-SSM, thereby forming an immobilised N-α-protected peptide fragment,
b) removing said N-α-protecting group, thereby forming an immobilised peptide fragment having an unprotected N-terminal,
c) coupling an additional amino acid or dipeptide having suitable protecting groups, including an N-α-protecting group, in the carboxyl activated form to the N-terminal of the immobilised peptide fragment, and repeating the removal/coupling step procedure in step b) and c) until the desired peptide sequence X is obtained, and then
d) coupling an additional amino acid or dipeptide having suitable protecting groups, including an N-α-protecting group, in the carboxyl activated form to the N-terminal of the immobilised peptide fragment, and repeating the removal/coupling step procedure in step b) and d) until the desired peptide sequence Z is obtained, and then
e) cleaving off the peptide conjugate from the solid support material.

The coupling, removal and cleavage steps are performed by methods known to the person skilled in the art taking into consideration the protection strategy and the selected solid phase material. In general, however, it is believed that the Boc (tert.butyloxycarbonyl) as well as the Fmoc (9-fluorenylmethyloxycarbonyl) protection strategies are applicable and that peptide bonds may be formed using the various activation procedures known to the person skilled in the art, e.g., by reacting a C-terminal activated derivative (acid halide, acid anhydride, activated ester e.g., HObt-ester, etc.) of the appropriate amino acid or peptide with the amino group of the relevant amino acid or peptide as known to a person skilled in peptide chemistry. Furthermore, it may be necessary or desirable to include side-chain protection groups when using amino acid residues carrying functional groups which are reactive under the prevailing conditions. The necessary protection scheme will be known to the person skilled in the art (cf., e.g., M. Bodanszky and A. Bodanszky, "The Practice of Peptide Synthesis", 2. Ed, Springer-Verlag, 1994, J. Jones, "The Chemical Synthesis of Peptides", Clarendon Press, 1991, and Dryland et al., 1986, J. Chem. Soc., Perkin Trans. 1:125-137).

The peptides and peptide conjugates of the invention may also be prepared by means of recombinant DNA technology using general methods and principles known to the person skilled in the art A nucleic acid sequence encoding the peptides and peptide conjugates may be prepared synthetically by established standard methods, e.g., the phosphoamidite method described by S.L. Beaucage and M.H. Caruthers, Tetrahedron Letters 22, 1981, pp. 1859-1869, or the method described by Matthes et al., EMBO Journal 3, 1984, pp. 801-805. According to the phosphoamidite method, oligonucleotides are synthesized, e.g., in an automatic DNA synthesizer, purified, annealed, ligated and cloned in suitable vectors. The techniques used to isolate or clone a nucleic acid sequence encoding peptide X are known in the art and include isolation from genomic DNA, preparation from cDNA, or a combination thereof. The cloning of the nucleic acid sequences of the present invention from such genomic DNA can be effected, e.g., by using the well known polymerase chain reaction (PCR) or antibody screening of expression libraries to detect cloned DNA fragments with shared structural features. See, e.g., Innis et al., 1990, A Guide to Methods and Application, Academic Press, New York. Other nucleic acid amplification procedures such as ligase chain reaction (LCR), ligated activated transcription (LAT) and nucleic acid sequence-based amplification (NASBA) may be used. It can then be ligated to a nucleic acid sequence encoding Z.

The nucleic acid sequence encoding the peptides and peptide conjugates is then inserted into a recombinant expression vector which may be any vector which may conveniently be subjected to recombinant DNA procedures. The choice of vector will often depend on the host cell into which it is to be introduced. Thus, the vector may be an autonomously replicating vector, i.e., a vector which exists as an extrachromosomal entity, the replication of which is independent of chromosomal replication, e.g., a plasmid. Alternatively, the vector may be one which, when introduced into a host cell, is integrated into the host cell genome and replicated together with the chromosome(s) into which it has been integrated.

In the vector, the nucleic acid sequence encoding the peptides and peptide conjugates of the present invention should be operably connected to a suitable promoter sequence. The promoter may be any nucleic acid sequence which shows transcriptional activity in the host cell of choice and may be derived from genes encoding proteins either homologous or heterologous to the host cell. Examples of suitable promoters for directing the transcription of the nucleic acid sequence encoding said peptides and peptide conjugates in mammalian cells are the SV 40 promoter (Subramani et al., Mol. Cell Biol. 1, 1981, pp. 854-864), the MT-1 (metallothionein gene) promoter (Palmiter et al., Science 222, 1983, pp. 809-814) or the adenovirus 2 major late promoter, a Rous sarcoma virus (RSV) promoter, cytomegalovirus (CMV) promoter (Boshart et al., 1981, Cell 41:521-530) and a bovine papilloma virus promoter (BPV). A suitable promoter for use in insect cells is the polyhedrin promoter (Vasuvedan et al., FEBS Lett. 311, 1992, pp. 7-11).

Examples of suitable promoters for directing the transcription of the nucleic acid sequence encoding the peptides and peptide conjugates, especially in a bacterial host cell, are the promoters obtained from the E. *coli* lac operon, the *Streptomyces coelicolor* agarase gene (dagA), the *Bacillus subtilis* levansucrase gene (sacB), the *Bacillus licheniformis* alpha-amylase gene (amyL), the *Bacillus stearothermophilus* maltogenic amylase gene (amyM), the *Bacillus amyloliquefaciens* alpha amylase gene (amyQ), the *Bacillus licheniformis* penicillinase gene (penP), the *Bacillus subtilis* xylA and xylB genes, and the prokaryotic beta-lactamase gene (Villa-Kamaroff et al., 1978, Proceedings of the National Academy of Sciences USA 75:3727-3731), as well as the tac promoter (DeBoer et al., 1983, Proceedings of the National Academy of Sciences USA 80:21 25). Further promoters are described in "Useful proteins from recombinant bacteria" in Scientific American, 1980, 242:74-94; and in Sambrook et al., 1989, supra. Examples of suitable promoters for directing the transcription of the nucleic acid sequence encoding the peptides and peptide conjugates in a filamentous fungal host cell are promoters obtained from the genes encoding *Aspergillus oryzae* TAKA amylase, *Rhizomucor miehei* aspartic proteinase, *Aspergillus niger* neutral alpha-amylase, *Aspergillus niger* acid stable alpha-amylase, *Aspergillus niger or Aspergillus awamori* glucoamylase (glaA), *Rhizomucor miehei* lipase, *Aspergillus oryzae* alkaline protease, *Aspergillus oryzae* triose phosphate isomerase, Aspergillus nidulans acetamidase, *Fusarium oxysporum* trypsin-like protease (as described in U.S. Patent No. 4,288,627, which is incorporated herein by reference), and hybrids thereof. Particularly preferred promoters for use in filamentous fungal host cells are the TAKA amylase, NA2-tpi (a hybrid of the promoters from the genes encoding *Aspergillus niger* neutral a amylase and *Aspergillus oryzae* triose phosphate isomerase), and glaA promoters. In a yeast host, useful promoters are obtained from the *Saccharomyces cerevisiae* enolase (ENO-1) gene, the *Saccharomyces cerevisiae* galactokinase gene (GAL1), the *Saccharomyces cerevisiae* alcohol dehydrogenase/glyceraldehyde-3-phosphate dehydrogenase genes (ADH2/GAP), and the *Saccharomyces cerevisiae* 3-phosphoglycerate kinase gene. Other useful promoters for yeast host cells are described by Romanos et al., 1992, Yeast 8:423-488.

The nucleic acid sequence encoding said peptides and peptide conjugates may also be operably connected to a suitable terminator, such as the human growth hormone terminator (Palmiter et al., op. cit.) Preferred terminators for filamentous fungal host cells are obtained from the genes encoding *Aspergillus oryzae* TAKA amylase, *Aspergillus niger* glucoamylase, *Aspergillus nidulans* anthranilate synthase, *Aspergillus niger* alpha-glucosidase, and *Fusarium oxysporum* trypsin-like protease. Preferred terminators for yeast host cells are obtained from the genes encoding *Saccharomyces cerevisiae* enolase, *Saccharomyces cerevisiae* cytochrome C (CYC1), or *Saccharomyces cerevisiae* glyceraldehyde-3-phosphate dehydrogenase. Other useful terminators for yeast host cells are described by Romanos et al., 1992, supra.

The vector may further comprise elements such as polyadenylation signals (e.g., from SV 40 or the adenovirus 5 Elb region), transcriptional enhancer sequences (e.g., the SV 40 enhancer) and translational enhancer sequences (e.g., the ones encoding adenovirus VA RNAs). Furthermore, preferred polyadenylation sequences for filamentous fungal host cells are obtained from the genes encoding *Aspergillus oryzae* TAKA amylase, *Aspergillus niger* glucoamylase, *Aspergillus nidulans* anthranilate synthase, and *Aspergillus niger* alpha-glucosidase. Useful polyadenylation sequences for yeast host cells are described by Guo and Sherman, 1995, Molecular Cellular Biology 15:5983-5990.

The recombinant expression vector may further comprise a DNA sequence enabling the vector to replicate in the host cell in question. Examples of such a sequence (when the host cell is a mammalian cell) is the SV 40 or polyoma origin of replication. Examples of bacterial origins of replication are the origins of replication of plasmids pBR322, pUC19, pACYC177, pACYC184, pUB110, pE194, pTA1060, and pAMβ1 . Examples of origin of replications for use in a yeast host cell are the 2 micron origin of replication, the combination of CEN6 and ARS4, and the combination of CEN3 and ARS1. The origin of replication may be one having a mutation to make its function temperature-sensitive in the host cell (see, e.g., Ehrlich, 1978, Proc. Natl. Acad. Sci. USA 75:1433).

The vector may also comprise a selectable marker, e.g., a gene the product of which complements a defect in the host cell, such as the gene coding for dihydrofolate reductase (DHFR) or one which confers resistance to a drug, e.g., neomycin, geneticin, ampicillin, or hygromycin. Suitable markers for yeast host cells are ADE2, HIS3, LEU2, LYS2, MET3, TRP1, and URA3. A selectable marker for use in a filamentous fungal host cell may be selected from the group including, but not limited to, amdS (acetamidase), argB (ornithine carbamoyltransferase), bar (phosphinothricin acetyltransferase), hygB (hygromycin phosphotransferase), niaD (nitrate reductase), pyrG (orotidine-5'-phosphate decarboxylase), sC (sulfate adenyltransferase), trpC (anthranilate synthase), and glufosinate resistance markers, as well as equivalents from other species. Preferred for use in an Aspergillus cell are the amdS and pyrG markers *ofAspergillus nidulans or Aspergillus oryzae* and the bar marker of *Streptomyces hygroscopicus.* Furthermore, selection may be accomplished by cotransformation, e.g., as described in WO 91/17243, where the selectable marker is on a separate vector.

The procedures used to ligate the nucleic acid sequences coding for the peptides and peptide conjugates, the promoter and the terminator, respectively, and to insert them into suitable vectors containing the information necessary for replication, are well known to persons skilled in the art (cf., for instance, Sambrook et al., op.cit.).

The host cell into which the expression vector is introduced may be any cell which is capable of producing the peptides and peptide conjugates and is may be a eukaryotic cell, such as invertebrate (insect) cells or vertebrate cells, e.g., *Xenopus laevis* oocytes or mammalian cells, in particular insect and mammalian cells. Examples of suitable mammalian cell lines are the COS (e.g., ATCC CRL 1650), BHK (e.g., ATCC CRL 1632, ATCC CCL 10) or CHO (e.g., ATCC CCL 61) cell lines.

Methods for transfecting mammalian cells and expressing DNA sequences introduced in the cells are described in e.g., Kaufman and Sharp, 1982, J. Mol. Biol. 159:601-621; Southern and Berg, 1982, J. Mol. Appl. Genet. 1:327-341; Loyter et al., 1982, Proc. Natl. Acad. Sci. USA 79:422-426; Wigler et al., 1978, Cell 14:725; Corsaro and Pearson, 1981, Somatic Cell Genetics 7:603, Graham and van der Eb, 1973, Virology 52:456; Fraley et al., 1980, JBC 225:10431; Capecchi, 1980, Cell 22:479; Wiberg et al., 1983,NAR 11:7287; and Neumann et ai., 1982, EMBO J. 1:841-845. The host cell may also be a unicellular pathogen, e.g., a prokaryote, or a non-unicellular pathogen, e.g., a eukaryote. Useful unicellular cells are bacterial cells such as gram positive bacteria including, but not limited to, a *Bacillus* cell, e.g., *Bacillus alkalophilus, Bacillus amyloliquefaciens, Bacillus brevis, Bacillus circulars, Bacillus coagulans, Bacillus lautus, Bacillus lentus, Bacillus licheniformis, Bacillus megaterium, Bacillus stearothermophilus, Bacillus subtilis,* and *Bacillus thuringiensis;* or a Streptomyces cell, e.g., *Streptomyces lividans* or *Streptomyces murinus,* or gram negative bacteria such as *E. coli* and *Pseudomonas* sp. In a preferred embodiment, the bacterial host cell is a *Bacillus lentus. Bacillus licheniformis, Bacillus stearothermophilus* or *Bacillus subtilis* cell. The transformation of a bacterial host cell may, for instance, be effected by protoplast transformation (see, e.g., Chang and Cohen, 1979, Molecular General Genetics 168:111-115), by using competent cells (see, e.g., Young and Spizizin, 1961, Journal of Bacteriology 81:823-829, or Dubnar and Davidoff Abelson, 1971, Journal of Molecular Biology 56:209-221), by electroporation (see, e.g., Shigekawa and Dower, 1988, Biotechniques 6:742-751), or by conjugation (see, e.g., Koehler and Thorne, 1987, Journal of Bacteriology 169:5771-5278). The host cell may be a fungal cell. The fungal host cell may also be a yeast cell. "Yeast" as used herein includes ascosporogenous yeast (Endomycetales), basidiosporogenous yeast, and yeast belonging to the Fungi Imperfecti (Blastomycetes).

The medium used to culture the cells may be any conventional medium suitable for growing mammalian cells, such as a serum-containing or serum-free medium containing appropriate supplements, or a suitable medium for growing insect, yeast or fungal cells. Suitable media are available from commercial suppliers or may be prepared according to published recipes (e.g,. in catalogues of the American Type Culture Collection).

Thus, the invention also relates to a method for producing the exendin variants and peptide conjugates of the invention having a natural polypeptide sequence, comprising
a) introducing a nucleic acid sequence encoding a polypeptide sequence comprising the peptide sequence of the exendin variant or the peptide conjugate of the invention and a selectable marker contained within a nucleic acid construct or a vector into a host cell to obtain a recombinant host cell;
b) selecting said recombinant host cell;
c) culturing said recombinant host cells under conditions permitting the production of said polypeptide sequence;
d) isolating said polypeptide sequence from the culture; and
e) optionally cleaving said polypeptide sequence using an appropriate protease to obtain said peptide conjugate.

The variants and peptide conjugates of the invention having a natural polypeptide sequence thus produced by the cells may then be recovered from the culture medium by conventional procedures including separating the host cells from the medium by centrifugation or filtration, precipitating the proteinaceous components of the supernatant or filtrate by means of a salt, e.g., ammonium sulphate, purification by a variety of chromatographic procedures, e.g., ion exchange chromatography, affinity chromatography, or the like. The lipophilic substituent(s) may be attached to the peptide of the present invention using procedures known in the art. In one embodiment, the lipophilic substituent may be attached by incorporating an amino acid with the lipophilic substituent already attached in the standard synthesis method (see, for example, synthesis of compound 7 in the Examples section). Alternatively, the substituent may be attached after the peptide has been synthesized and isolated as, for example, described in WO98/08871.

The invention is further illustrated by the following examples.

### EXAMPLES

### Peptide Synthesis, General Procedures

- Apparatus and synthetic strategy
   Peptides are synthesized batchwise in a polyethylene vessel equipped with a polypropylene filter for filtration using 9-fluorenylmethyloxycarbonyl (Fmoc) as the N-α-amino protecting group and suitable common protection groups for side-chain functionalities (Dryland et al., 1986, J. Chem. Soc., Perkin Trans. 1:125-137).
- Solvents
   Solvent DMF (*N,N*-dimethylformamide, Riedel de-Häen, Germany) is purified by passing it through a column packed with a strong cation exchange resin (Lewatit S 100 MB/H strong acid, Bayer AG Leverkusen, Germany) and analysed for free amines prior to use by addition of 3,4-dihydro-3-hydroxy-4-oxo-1,2,3-benzotriazine (Dhbt-OH) giving rise to a yellow color (Dhbt-O-anion) if free amines are present. Solvent DCM (dichloromethane, analytical grade, Riedel de-Hätn, Germany) is used directly without purification. THF (tetrahydrofuran, analytical grade, Riedel de-Haen, Germany) is used directly without further purification.
- Amino acids
   Fmoc-protected amino acids are purchased from MilliGen (UK) and from PerSeptive Biosystems GmbH Hamburg, Germany in suitable side-chain protected forms. FmocLys(palmitoyl)-OH is purchased from Bachem (Switzerland).
- Linker
   (4-hydroxymethylphenoxy)acetic acid (HMPA), Novabiochem, Switzerland is coupled to the resin either as a preformed or *in situ* generated 1-hydroxybenzotriazole (HObt) ester by means of DIC.
- Coupling reagents
   Coupling reagent diisopropylcarbodiimide (DIC) is purchased from (Riedel de-Häen, Germany) and distilled prior to use, dicyclohexylcarbodiimide (DCC) is purchased from Merck-Schuchardt, München, Germany, and purified by distillation.
- Solid supports
   Peptides synthesized according to the Fmoc-strategy are synthesized on the following types of solid support using 0.05 M or higher concentrations of Fmoc-protected activated amino acid in DMF. TentaGel S resins 0.22-0.31 mmol/g (TentaGel S-Ram, TentaGel S RAM-Lys(Boc)Fmoc; Rapp polymers, Germany).
- Catalysts and other reagents
   Diisopropylethylamine (DIEA) is purchased from Aldrich, Germany, and ethylenediamine from Fluka, piperidine and pyridine from Riedel-de Häen, Frankfurt, Germany. 4 (N,N-dimethylamino)pyridine (DMAP) is purchased from Fluka, Switzerland and used as a catalyst in coupling reactions involving symmetrical anhydrides. Ethanedithiol is purchased from Riedel-de Häen, Frankfurt, Germany. 3,4-dihydro-3-hydroxy-4-oxo-21,2,3-benzotriazine (Dhbt-OH) and 1-hydroxybenzotriazole (HObt) are obtained from Fluka, Switzerland.
- Coupling procedures
   The first amino acid is coupled as a symmetrical anhydride in DMF generated from the appropriate N-α-protected amino acid by means of DIC or DCC. The following amino acids are coupled as preformed HObt esters made from appropriate N-α-protected amino acids and HObt by means of DIC in DMF. Acylations are checked by the ninhydrin test performed at 80°C in order to prevent Fmoc deprotection during the test (Larsen, B. D. and Holm, A., 1994, Int J. Peptide Protein Res. 43:1-9).
- Coupling as HObt-ester
   *Method a.* 3 eq. N-α-amino protected amino acid is dissolved in DMF together with 3 eq. HObt and 3 eq DIC. The solution is left at r.t. for 10 minutes and then added to the resin, which had been washed with a solution of 0.2% Dhbt-OH in DMF prior to the addition of the preactivated amino acid.
   *Method b.* 3 eq. N-α-amino protected amino acid is dissolved in DMF together with 3 eq. HObt. 3 eq DIC are added just prior to use. The final solution is added to the resin.
- Preformed symmetrical anhydride
   6 eq. N-α-amino protected amino acid is dissolved in DCM and cooled to 0°C. DCC or DIC (3 eq.) is added and the reaction continued for 10 min. The solvent is removed *in vacuo* and the residue dissolved in DMF. The DMF-solution is filtered in case of using DCC and immediately added to the resin followed by 0.1 eq. of DMAP.
- Deprotection of the N-α-amino Fmoc protecting group
   Deprotection of the Fmoc group is performed by treatment with 20% piperidine in DMF (1 x 5 and 1 x 10 min.), followed by wash with DMF until no yellow colour (Dhbt-O-) could be detected after addition of Dhbt-OH to the drained DMF.
- Cleavage of peptide from resin with acid
   *Method a.* Peptides are cleaved from the resins by treatment with 95% trifluoroacetic acid (TFA, Riedel-de Häen, Frankfurt, Germany)-water v/v or with 95% TFA and 5% ethanedithiol v/v at r.t. for 2 h. The filtered resins are washed with 95% TFA-water and filtrates and washings are diluted by adding 10% acetic acid. The resulting mixture is extracted 3 times with ether and finally freeze dried. The crude freeze dried product is analysed by high-performance liquid chromatography (HPLC) and identified by mass spectrometry (MS).
- Batchwise peptide synthesis on TentaGel S-RAM
   TentaGel S-RAM resin (100-1000 mg, 0.22-0.31 mmol/g) is placed in a polyethylene vessel equipped with a polypropylene filter for filtration. The resin is swelled in DMF (5-10 ml), and the Fmoc group is removed according to the procedure described above. The following amino acids according to the sequence are coupled as Fmoc-protected HObt esters (3 eq.) generated *in situ* by means of DIC as described above. The couplings are continued for 3 h, unless otherwise specified. The resin is drained and washed with DMF (4 x 5-10 ml, 2 min each) in order to remove excess reagent. All acylations are checked by the ninhydrin test performed at 80°C. After completion of the synthesis, the peptide-resin is washed with DMF (3 x 5-10 ml, 5 min each), DCM (3 x 5-10 ml, 1 min each) and finally diethyl ether (3 x 5-10 ml, 1 min each) and dried *in vacuo*.
- HPLC conditions
   Isocratic HPLC analysis is preformed on a Shimadzu system consisting of an LC-6A pump, an MERCK HITACHI L-4000 UV detector operated at 215 nm and a Rheodyne 7125 injection valve with a 20 µl loop. The column used for isocratic analysis is a Spherisorb ODS-2 (100 x 3 mm; 5-µm particles) (MicroLab, Aarhus, Denmark). HPLC analysis using gradients is performed on a MERCK-HITACHI L-6200 Intelligent pump, an MERCK HITACHI L-4000 UV detector operated at 215 nm and a Rheodyne 7125 injection valve with a 20 µl loop, or on a Waters 600 E instrument equipped with a Waters 996 photodiode array detector. The columns used are a Rescorce^{TM} RPC 1 ml (Waters) or a LiChroCART 125-4, LiChrospher 100 RP-18 (5 µm) (Merck).
   Buffer A is 0.1 vol % TFA in water and buffer B 90 vol% acetonitrile, 9.9 vol% water and 0.1 vol% TFA. The buffers are pumped through the columns at a flow rate of 1.3-1.5 ml/min using either of the following gradients for peptide analysis 1) Linear gradient from 0% - 100% B (30 min) or 2) 0% B (2 min) linear gradient from 0-50% B (23 min) 50-100% B (5 min).
   For Preparative HPLC, purification is performed on a Waters 600 E instrument equipped with a Waters 996 photodiode array detector. The column used is a Waters Delta-Pak C-18 15 µm, 100 Å, 25 x 100 mm. Gradient "2)" is used with a flow rate of 9 ml/min.
- Mass spectroscopy
   Mass spectra are obtained on a Finnigan Mat LCQ instrument equipped with an electrospray (ESI) probe (ES-MS) and on a TofSpec E, Fisons Instrument (MALDI-TOF) using β-cyano-*p*-hydroxycinnamic acid as matrix. Alternatively, spectra may be obtained by a Micromass LCT instrument.

### Peptide synthesis of prior art peptides

(i) Peptide synthesis of Compound (i). H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH₂
   (exendin-4(1-39)-NH₂) (SEQ ID NO:102) on TentaGel S-RAM.
   Dry TentaGel S-RAM resin (0.25 mmol/g, 1000 mg) is placed in a polyethylene vessel equipped with a polypropylene filter for filtration and swelled for two hours in DMF (5 ml). The Fmoc group is removed according to the procedure described above, and the peptide according to the sequence is assembled as described under "Batchwise peptide synthesis on TentaGel S-RAM resins". After completion of the synthesis, the peptide-resin is washed with DMF (3x5 ml, 1 min each), DCM (3x5 ml, 1 min each), diethyl ether (3x5 ml, 1 min each) and dried *in vacuo.* The peptide is cleaved from the resin according to *method a* as described above and freeze dried from acetic acid. The crude peptide is purified by preparative HPLC using the procedure described above. The purified product is found to be homogeneous and the purity is found to be better than 90%. The identity of the peptide is confirmed by ES-MS. Yield 17%.
(ii) Peptide synthesis of Compound (ii), H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-NH₂
   (des Ser³⁹ exendin-4(1-39)-NH₂) on TentaGel S-RAM.
   Dry TentaGel S-RAM resin (0.25 mmol/g, 1000 mg) is placed in a polyethylene vessel equipped with a polypropylene filter for filtration and swelled for two hours in DMF (5 ml). The Fmoc group is removed according to the procedure described above, and the peptide according to the sequence is assembled as described under "Batchwise peptide synthesis on TentaGel S-RAM resins". After completion of the synthesis, the peptide-resin is washed with DMF (3x5 ml, 1 min each), DCM (3x5 ml, 1 min each), diethyl ether (3x5 ml, 1 min each) and dried *in vacuo.* The peptide is cleaved from the resin according to *method a* as described above and freeze dried from acetic acid. The crude peptide is purified by preparative HPLC using the procedure described above. The purified product is found to be homogeneous and the purity is found to be better than 97%. The identity of the peptide is confirmed by ES-MS. Yield 22%.

### 1. Peptide synthesis of Compound 1, H-His-Gly-Glu-Gly-Thr-Phe-Thr-Ser-Asp-Leu-Ser-Lys-GIn-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Ser-NH₂ (des Pro³⁶-exendin-4(1-39)-NH₂) (SEQ ID NO:101) on TentaGel S-RAM.

Dry TentaGel S-RAM resin (0.25 mmol/g, 1500 mg) is placed in a polyethylene vessel equipped with a polypropylene filter for filtration and swelled for two hours in DMF (5 ml). The Fmoc group is removed according to the procedure described above, and the peptide according to the sequence is assembled as described under "Batchwise peptide synthesis on TentaGel S-RAM resins". After completion of the synthesis, the peptide-resin is washed with DMF (3x5 ml, 1 min each), DCM (3x5 ml, 1 min each), diethyl ether (3x5 ml, 1 min each) and dried *in vacuo.* The peptide is cleaved from the resin according to *method a* as described above and freeze dried from acetic acid. The crude peptide is purified by preparative HPLC using the procedure described above. The purified product is found to be homogeneous and the purity is found to be better than 95%. The identity of the peptide is confirmed by ES-MS. Yield 18.3%.

### 2. Peptide synthesis of Compound 2, H-His-Giy-Glu-Giy-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Ser-(Lys)₆-NH₂ (des-Pro³⁶-exendin-4(1-39)-Lys₆-NH₂) (SEQ ID NO:93) on TentaGel S-RAM-Lys(Boc)Fmoc.

Dry TentaGel S-RAM-Lys(Boc)Fmoc resin (0.22 mmol/g, 1500 mg) is placed in a polyethylene vessel equipped with a polypropylene filter for filtration and swelled for two hours in DMF (5 ml). The Fmoc group on the first lysine is removed as described above and the synthesis is continued until finishing the peptide sequence as described under "Batchwise peptide synthesis on TentaGel S-Ram-Lys(Boc)Fmoc". After completion of the synthesis, the peptide-resin is washed with DMF (3x5 ml, 1 min each), DCM (3x5 ml, 1 min each), diethyl ether (3x5 ml, 1 min each) and dried *in vacuo.* The peptide is cleaved from the resin according to *method a* as described above and freeze dried from acetic acid. The crude freeze dried product is purified by preparative HPLC using the procedure described above. The purified product is found to be homogeneous and the purity is found to be better than 95%. The identity of the peptide is confirmed by ES-MS. Yield 22.1%.

### 3. Peptide synthesis of Compound 3, H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser(Lys)₆-NH₂ (exendin-4(1-39)-Lys₆-NH₂) (SEQ ID NO:92) on TentaGel S-RAM-Lys(Boc)Fmoc.

Dry TentaGel S-RAM-Lys(Boc)Fmoc resin (0.22 mmol/g, 1000 mg) is placed in a polyethylene vessel equipped with a polypropylene filter for filtration and swelled for two hours in DMF (5 ml). The Fmoc group on the first lysine is removed as described above and the synthesis is continued until finishing the peptide sequence as described under "Batchwise peptide synthesis on TentaGel S-Ram-Lys(Boc)Fmoc". After completion of the synthesis, the peptide-resin is washed with DMF (3x5 ml, 1 min each), DCM (3x5 ml, 1 min each), diethyl ether (3x5 ml, 1 min each) and dried *in vacuo.* The peptide is cleaved from the resin according to *method a* as described above and freeze dried from acetic acid. The crude freeze dried product is purified by preparative HPLC using the procedure described above. The purified product is found to be homogeneous and the purity is found to be better than 90%. The identity of the peptide is confirmed by ES-MS. Yield 20.5%.

### 4. Peptide synthesis of Compound 8, H-Lys-Lys-Lys-Lys-Lys-Lys-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Ser-NH₂ (H-(Lys)₆-des Pro³⁶exendin-4(1-39)-NH₂) on TentaGel S-RAM-Fmoc.

Dry TentaGel S-RAM-Fmoc resin (0.23 mmol/g, 1000 mg) is placed in a poly-ethylene vessel equipped with a polypropylene filter for filtration and swelled for two hours in DMF (5 ml). The Fmoc group on the resin is removed as described above and the synthesis is continued until finishing the peptide sequence as described under "Batchwise peptide synthesis on TentaGel S-Ram-Fmoc". After completion of the synthesis, the peptide-resin is washed with DMF (3x5 ml, 1 min each), DCM (3x5 ml, 1 min each), diethyl ether (3x5 ml, 1 min each) and dried in vacuo. The peptide is cleaved from the resin according to method a as described above and freeze dried from acetic acid. The crude freeze dried product is purified by preparative HPLC using the procedure described above. The purified product is found to be homogeneous and the purity is found to be better than 95%. The identity of the peptide is confirmed by ES-MS. Yield 26%.

### 5. Peptide synthesis of Compound 9, H-Lys₆-His-gly-Glu-Gly-Thr-Phe-Thr-Ser-Asp-Leu-Ser-Lys-Gln-Met-Glu-Glu-Glu-Ala-Vat-Arg-Leu-Phe-Ile-Glu-Trp-Leu-Lys-AsnÆlynly-Pro Ser-Ser-Gly-Ala-Pro-Pro-Ser-(Lys)₆-NH₂ (H-Lys₆-des Pro³⁶exendin-4(1-39)-Lys₆-NH₂) on TentaGel S-RAM-Lys(Boc)Fmoc.

Dry TentaGel S-RAM-Lys(Boc)Fmoc resin (0.22 mmol/g, 1000 mg) is placed in a poly-ethylene vessel equipped with a polypropylene filter for filtration and swelled for two hours in DMF (5 ml). The Fmoc group on the first lysine is removed as described above and the synthesis is continued until finishing the peptide sequence as described under "Batchwise peptide synthesis on TentaGel S-Ram-Lys(Boc)Fmoc". After completion of the synthesis, the peptide-resin is washed with DMF (3x5 ml, 1 min each), DCM (3x5 ml, 1 min each), diethyl ether (3x5 ml, 1 min each) and dried in vacuo. The peptide is cleaved from the resin according to method a as described above and freeze dried from acetic acid. The crude freeze dried product is purified by preparative HPLC using the procedure described above. The purified product is found to be homogeneous and the purity is found to be better than 90%. The identity of the peptide is confirmed by ES-MS. Yield 32%.

### 6. Peptide synthesis of Compound 14. H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Ser-NH₂ (H-des Pro³⁶, Pro³⁷, Pro³⁸exendin-4(1-39)-NH₂) on TentaGel S-RAM-Fmoc.

Dry TentaGel S-RAM-Fmoc resin (0.23 mmol/g, 1000 mg) is placed in a poly-ethylene vessel equipped with a polypropylene filter for filtration and swelled for two hours in DMF (5 ml). The Fmoc group on the resin is removed as described above and the synthesis is continued until finishing the peptide sequence as described under "Batchwise peptide synthesis on TentaGel S-Ram-Fmoc". After completion of the synthesis, the peptide-resin is washed with DMF (3x5 ml, 1 min each), DCM (3x5 ml, 1 min each), diethyl ether (3x5 ml, 1 min each) and dried in vacuo. The peptide is cleaved from the resin according to method a as described above and freeze dried from acetic acid. The crude freeze dried product is purified by preparative HPLC using the procedure described above. The purified product is found to be homogeneous and the purity is found to be better than 95%. The identity of the peptide is confirmed by ES-MS. Yield 11%.

### 7. Peptide synthesis of Compound 15. H-Lys-Lys-Lys-Lys-Lys-Lys-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Ser-NH₂ (H-(Lys)₆-des Pro³⁶, Pro³⁷, Pro³⁸exendin-4(1-39)-NH₂) on TentaGel S-RAM-Fmoc.

Dry TentaGel S-RAM-Fmoc resin (0.23 mmol/g, 1000 mg) is placed in a poly-ethylene vessel equipped with a polypropylene filter for filtration and swelled for two hours in DMF (5 ml). The Fmoc group on the resin is removed as described above and the synthesis is continued until finishing the peptide sequence as described under "Batchwise peptide synthesis on TentaGel S-Ram-Fmoc". After completion of the synthesis, the peptide-resin is washed with DMF (3x5 ml, 1 min each), DCM (3x5 ml, 1 min each), diethyl ether (3x5 ml, 1 min each) and dried in vacuo. The peptide is cleaved from the resin according to method a as described above and freeze dried from acetic acid. The crude freeze dried product is purified by preparative HPLC using the procedure described above. The purified product is found to be homogeneous and the purity is found to be better than 94%. The identity of the peptide is confirmed by ES-MS. Yield 17%.

### 8. Peptide synthesis of compound 16. H-Asn-Glu-Glu-Glu-Glu-Glu-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Ser-NH₂ (H-Asn-(Glu)₅-des Pro³⁶, Pro³⁷, Pro³⁸exendin-4(1-39)NH²) on TentaGel S-RAM-Fmoc.

Dry TentaGel S-RAM-Fmoc resin (0.23 mmol/g, 1000 mg) is placed in a poly-ethylene vessel equipped with a polypropylene filter for filtration and swelled for two hours in DMF (5 ml). The Fmoc group on the resin is removed as described above and the synthesis is continued until finishing the peptide sequence as described under "Batchwise peptide synthesis on TentaGel S-Ram-Fmoc". After completion of the synthesis, the peptide-resin is washed with DMF (3x5 ml, 1 min each), DCM (3x5 ml, 1 min each), diethyl ether (3x5 ml, 1 min each) and dried in vacuo. The peptide is cleaved from the resin according to method a as described above and freeze dried from acetic acid. The crude freeze dried product is purified by preparative HPLC using the procedure described above. The purified product is found to be homogeneous and the purity is found to be better than 90%. The identity of the peptide is confirmed by ES-MS. Yield 9%.

### 9 Peptide synthesis of Compound 17, Compound 3, H-(Lys)₆-His-Gly-Glu-Gly-Thr-Phe-Thr-Ser-Asp-Leu-Ser-Lys-Gln-Met-GIu-Glu-Glu-Ala-Val-Arg-Leu-Phe-Ile-Glu-Trp-Leu-Lys-Asn-Gly-Gly-Pro-Ser-Ser-Gly-Ala-Ser-(Lys)₆-NH₂ (H-(Lys)₆-des Pro³⁶, Pro³⁷, Pro³⁸exendin-4(1-39)-(Lys)₆-NH₂) on TentaGel S-RAM-Lys(Boc)Fmoc.

Dry TentaGel S-RAM-Lys(Boc)Fmoc resin (0.22 mmol/g, 1000 mg) is placed in a poly-ethylene vessel equipped with a polypropylene filter for filtration and swelled for two hours in DMF (5 ml). The Fmoc group on the first lysine is removed as described above and the synthesis is continued until finishing the peptide sequence as described under "Batchwise peptide synthesis on TentaGel S-Ram-Lys(Boc)Fmoc". After completion of the synthesis, the peptide-resin is washed with DMF (3x5 ml, 1 min each), DCM (3x5 ml, 1 min each), diethyl ether (3x5 ml, 1 min each) and dried in vacuo. The peptide is cleaved from the resin according to method a as described above and freeze dried from acetic acid. The crude freeze dried product is purified by preparative HPLC using the procedure described above. The purified product is found to be homogeneous and the purity is found to be better than 90%. The identity of the peptide is confirmed by ES-MS. Yield 10%.

### 10. Peptide synthesis of Compound 18, H-Asn-Glu-Glu-Glu-Glu-Glu-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Ser-(Lys)₆-NH₂ (H-Asn-(Glu)₅-des Pro³⁶, Pro³⁷, Pro³⁸exendin-4(1-39)-(Lys)₆-NH₂) on TentaGel S-RAM-Lys(Boc)Fmoc.

Dry TentaGel S-RAM-Lys(Boc)Fmoc resin (0.22 mmol/g, 1000 mg) is placed in a poly-ethylene vessel equipped with a polypropylene filter for filtration and swelled for two hours in DMF (5 ml). The Fmoc group on the first lysine is removed as described above and the synthesis is continued until finishing the peptide sequence as described under "Batchwise peptide synthesis on TentaGel S-Ram-Lys(Boc)Fmoc". After completion of the synthesis, the peptide-resin is washed with DMF (3x5 ml, 1 min each), DCM (3x5 ml, 1 min each), diethyl ether (3x5 ml, 1 min each) and dried in vacuo. The peptide is cleaved from the resin according to method a as described above and freeze dried from acetic acid. The crude freeze dried product is purified by preparative HPLC using the procedure described above. The purified product is found to be homogeneous and the purity is found to be better than 92%. The identity of the peptide is confirmed by ES-MS. Yield 14%.

### 11. Peptide synthesis of Compound 19, H-His-Gly-Glu-Gly-Thr-Phe-Thr-Ser-Asp-Leu-Ser-Lys-Gin-Met-Glu-Glu-Glu-Ala-Val-Arg-Leu-Phe-IIe-Glu-Trp-Leu-Lys-Asn-Gly-Gly-Pro-Ser-Ser-Gly-Ala-Ser-(Lys)₆-NH₂ (des Pro³⁶, Pro³⁷, Pro³⁸exendin-4(1-39)-(Lys)₆-NH₂) on TentaGel S-RAM-Lys(Boc)Fmoc.

Dry TentaGel S-RAM-Lys(Boc)Fmoc resin (0.22 mmol/g, 1000 mg) is placed in a poly-ethylene vessel equipped with a polypropylene filter for filtration and swelled for two hours in DMF (5 ml). The Fmoc group on the first lysine is removed as described above and the synthesis is continued until finishing the peptide sequence as described under "Batchwise peptide synthesis on TentaGel S-Ram-Lys(Boc)Fmoc". After completion of the synthesis, the peptide-resin is washed with DMF (3x5 ml, 1 min each), DCM (3x5 ml, 1 min each), diethyl ether (3x5 ml, 1 min each) and dried in vacuo. The peptide is cleaved from the resin according to method a as described above and freeze dried from acetic acid. The crude freeze dried product is purified by preparative HPLC using the procedure described above. The purified product is found to be homogeneous and the purity is found to be better than 97%. The identity of the peptide is confirmed by ES-MS. Yield 19%.

### 12. Recombinant preparation of Compound 2

### Construction of the pYES0010 expression vector

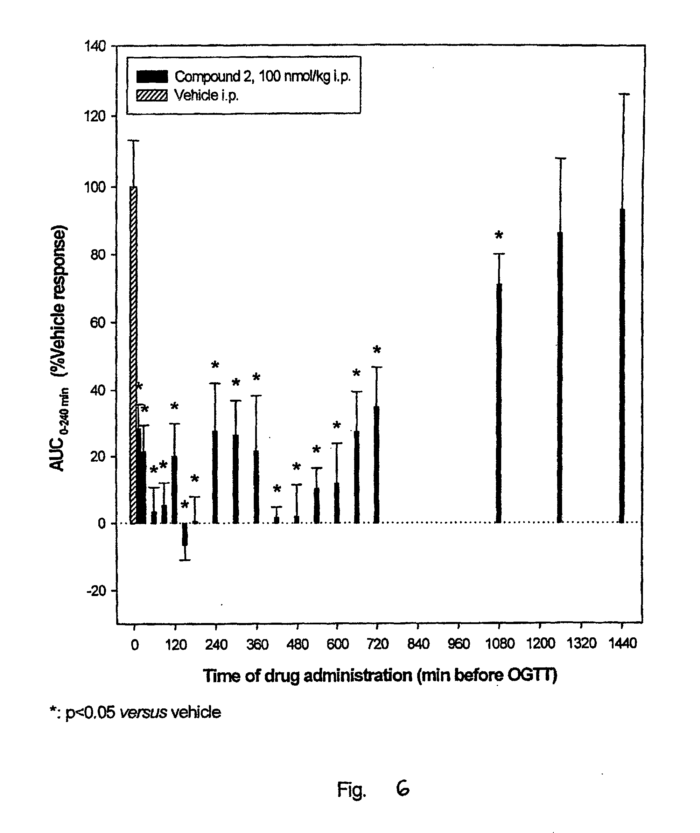

A synthetic cDNA was constructed for heterolog expression in yeast. The protein sequence encoding Compound 2 was back translated using a Saccharomyces cerevisiae codon usage table (Saccharomyces Genome Database). To enable translation of the synthetic cDNA an additional ATG start codon was added to the 5' end and a TAA stop codon was added to the 3' end. The construct was inserted into HindIII and The EcoRI site of the pYES2 shuttle vector comprising an ampicilline resistance gene, and the new construct was designated pYES0010, cf. Fig. 6. pYES0010 was subsequently transformed into E. coli and subjected to ampicillin selection pressure. Positive clones were selected and sequenced.

### Transformation into yeast.

In order to make transform the pYES0010 into the yeast haploid INVSc1: MATa his3delta1 leu2 trp1-289 ura3-52. Yeast were grown in YPD medium (1% yeast extract, 2 % peptone, 2% glucose, and 0.004% adenine sulfate) at 30 C to saturation. 1ml of culture was harvested for transformations. 2 µl of 10 mg/ml carrier DNA was added and 1 µg of pYES0010 was added and mixed. 0.5 ml (45% PEG 4000,1M Li OAc, 0.5M EDTA and 1M Tris-HCl (pH 7.5) was added and mixed. Finally 20µl 1M DTT was added and the mixture was incubated for 16 h at room temperature. After incubation the cells were heat shocked at 42 C for 10 min and plated selective plates ( 6.7 % yeast nitrogen base, 2% glucose, 20µg/ml adenine, 20µg/ml arginine, 29µg/ml isoleucine, 20µg/ml histidine, 60µg/ml leucine, 20µg/ml lysine,20µg/ml tryptophan, 20µg/ml methionine 50µg/ml phenylalanine 150 µg/ml valine, 30µg/ml Tyrosine and 2.5% agar. Plates were incubated at 30 C for 3 to 5 days until transformants appear.

### Expression and purification of Compound 2.

Transformants were cultivated in selective media (6.7 % Yeast nitrogen base, 2% glucose, 20µg/ml adenine, 20µg/ml arginine, 29µg/ml isoleucine, 20µg/ml histidine, 60µg/ml leucine, 20µg/ml lysine, 20µg/ml Tryptophan, 20µg/ml methionine 50µg/ml phenylalanine 150 µg/ml valine, 30µg/ml Tyrosine) for 1.5 days. The cells were harvested and resuspended in galactose induction medium (6.7 % Yeast nitrogen base, 4% galactose, 20µg/ml adenine, 20µg/ml arginine, 29µg/ml isoleucine, 20µg/ml histidine, 60µg/ml leucine, 20µg/ml lysine, 20µg/ml Tryptophan, 20µg/ml methionine 50µg/ml phenylalanine 150 µg/ml valine, 30µg/ml Tyrosine for 1 day. The cells were harvested and homogenized in 10mM Tris-HCl pH 7.5 containing protease inhibitors (Roche). The lysate was clarified centrifugation at 20.000 X g for 30 min. The supernatant was loaded onto a Superdex 12 HR 10/30 column (Amersham Pharmacia Biotech) equilibrated with 10mM Tris-HCl pH 7.5. The column was eluted in 50Mm ammonia bicarbonate buffer pH 8.0. Samples containing recombinant Compound 2 were pooled. The N-terminal methionine was removed by methionine aminopeptidase and the samples were further purified on a HPLC Column.

### HPLC Settings for Compound 2 purification.

- HPLC column :: Kromasil RP C8; K 100-10-C8 nr. CER 2230. compound
- Temp:: 22 C
- Flow rate:: 35ml/min

### HPLC solvents:

- A:: 0.10% trifluoroacetic acid in water
- B:: 0.10% trifluoroacetic acid in acetonitrile: water 90:10.

Compound 2 was eluted from the HPLC column with 0.10% trifluoroacetic acid in 20% to 80% Acetonitrile in 40 min.

### 13. Injection formulations of peptide

Fixed dose formulations of peptide for intra venous injection are prepared by dissolving the peptide in sterile, isotonic saline, and storing the resulting solution in glass ampoules filled with inert gas under sterile conditions. Each dose of the peptide is stored dry in ampoules or capped vials filled with inert gas. Multi-dose formulations of peptide for intra venous injection are prepared by dissolving the peptide in sterile, isotonic saline, storing the resulting solution in capped vials, if necessary adding preservative (for instance 0.1 % parahydroxybenzoate, 1 % benzyl alcohol or 0.1 % chlorocresole).

| Example of multi-dose peptide formulation: | |
|---|---|
| Compound 2 | 12.25 mg |
| Sodiumdihydrogenphosphate | 1.380 g |
| Parahydroxybenzoate | 0.1 g |
| Aqua ad injectabile | 100 ml |

### 14. Single Dose Effect of oral and parenteral administration of Compounds on Blood Glucose Levels in Diabetic ob/ob Mice.

The compounds of the invention possess blood glucose lowering properties. This effect of compounds was examined by testing their effect on blood glucose (BG) levels in the ob/ob mutant mice after intraperitoneal (i.p.) and peroral (p.o.) administration.

### Experimental

Forty female diabetic ob/ob mice (Umea strain, Bomholtgaard), which are obese due to a dominant mutant leptin (Tomita, T., Doull, V., Pollock, H. G., and Krizsan, D. 1992. Pancreatic islets of obese hyperglycemic mice (ob/ob). Pancreas 7: 367-75) were housed (3 mice/cage) under controlled ambient conditions following a 12:12-h light:dark cycle and fed standard Altromin no 1324 diet with free access to tap water. At arrival the animals were 8 weeks of age. The mice were allowed 2 weeks of acclimatization before experiments were initiated. At the time of experiment the mice were 13 weeks old with a body weight of 41.8± 3.2 g (mean ± SD; n=42). Handling of the mice one and three days before the experiment was performed in order to reduce stress-induced BG excursions. On the day of the experiment, blood was taken from the tip of the tail 2-3 hours after the light was turned on. A single drop of blood (<5 µl) was dropped on the glucose strip for analysis and measured by an Elite Autoanalyser, Bayer, Denmark. Whole blood glucose (BG) concentration was analysed by the immobilised glucose oxidase method. Blood glucose levels varied between normoglycaemia and severe hyperglycaemia (range: 3.6-15.6 mM; mean ± SD: 9.4 ± 3.3 mM; n=42). Six animals with BG < 5.8 mM were excluded from the study (total n=36). The remaining animals were stratified based on their BG levels in order to ensure that the mean BG was similar among groups. One hour after the initial control blood sampling, drugs were administered and BG was measured at t=60 min, t=120 min, t=240 min,t=480 min.

### Peptides and other materials

The compound was dissolved in sterile isotonic NaCl shortly before dosing and given in a volume of 0.2 ml. The same solutions were used for both p.o. and i.p. administration. For each animal, a data log sheet was filled out at the time of each blood sampling.

### 15. In vivo Studies with

Compound 1 (des Pro³⁶-exendin-4(1-39)-NH₂ (SEQ ID NO:101)),
Compound 2 (des Pro³⁶-exendin-4(1-39)-Lys6-NH₂ (SEQ ID NO:93)),

Various concentrations of each peptide are administered orally and intraperitoneally to *oblob* mice to determine if these compounds affect blood glucose levels. The experimental conditions used were the same as described in Example 14.

### Peptides and other materials

Des Pro³⁶-exendin-4(1-39)-NH₂ (Compound 1, SEQ ID NO:101) and the same peptide, but with an additional sequence, Lys_{6,} attached at the C-terminal, des Pro³⁶-exendin-4(1-39)-Lys₆-NH₂ (Compound 2, SEQ ID NO:93), Gly⁸-GLP1-(7-36)(Human)-NH₂ (Compound (ii), SEQ ID NO:87)and the same peptide, but with an additional sequence, Lys_{6,} attached at the C-terminal, are synthesized using methods described above. Solutions are prepared on the morning of dosing, immediately before the animals are administered. The same solutions are used for both peroral and interperitoneal administration. All peptides are dissolved in sterile isotonic Nacl and given in a volume of 0.2 ml. All experiments are carried out in the same mice to compare the active doses of the peptides shown in Table 1. Blood sampling is performed as described above and the animals are administered with the doses shown in Table 2. As negative control, saline is administered perorally. Results are shown in Table 3 .

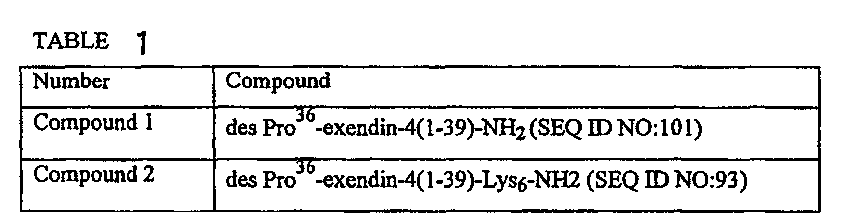

**TABLE 2**

| Compound | Group 1 Dose peroral µg/mouse | Group 2 Dose peroral µg/mouse | Group 3 Dose peroral µg/mouse | Group 4 Dose peroral µg/mouse | Group 5 Dose peroral µl/mouse Isotonic saline | Group 6 Dose i.p µg/mouse |
|---|---|---|---|---|---|---|
| Compound 1 | 400 | 40 | 4 | 0.4 | 200 µl | 40 |
| Compound 2 | 1000 | 100 | 10 | 1 | 200 µl | 100 |

Group data were summarised as the mean ± SEM of the individual results in each treatment group. In order to analyse the effects of the compounds, the absolute and the relative (% of t = 0) difference from baseline was calculated for each time point.

**TABLE 3**

| | 0 | 1 hour | 2 hours | 4 hours |
|---|---|---|---|---|
| Compound 1-saline | 100 | 103 | 107 | 92 |
| Compound 1-400 µg po | 100 | 93 | 88 | 93 |
| Compound 1-40 µg po | 100 | 89 | 89 | 91 |
| Compound 1-4 µg po | 100 | 105 | 88 | 91 |
| Compound 1-0.4 µg po | 100 | 106 | 103 | 100 |
| Compound 1-40 µg ip | 100 | 68 | 69 | 74 |
| Compound 2-saline | 100 | 100 | 112 | 114 |
| Compound 2-1000 µg po | 100 | 67 | 69 | 64 |
| Compound 2 100 µg po | 100 | 78 | 71 | 72 |
| Compound 2 10 µg po | 100 | 86 | 72 | 72 |
| Compound 2 1 µg po | 100 | 112 | 101 | 96 |
| Compound 2 100 µg ip | 100 | 75 | 67 | 63 |

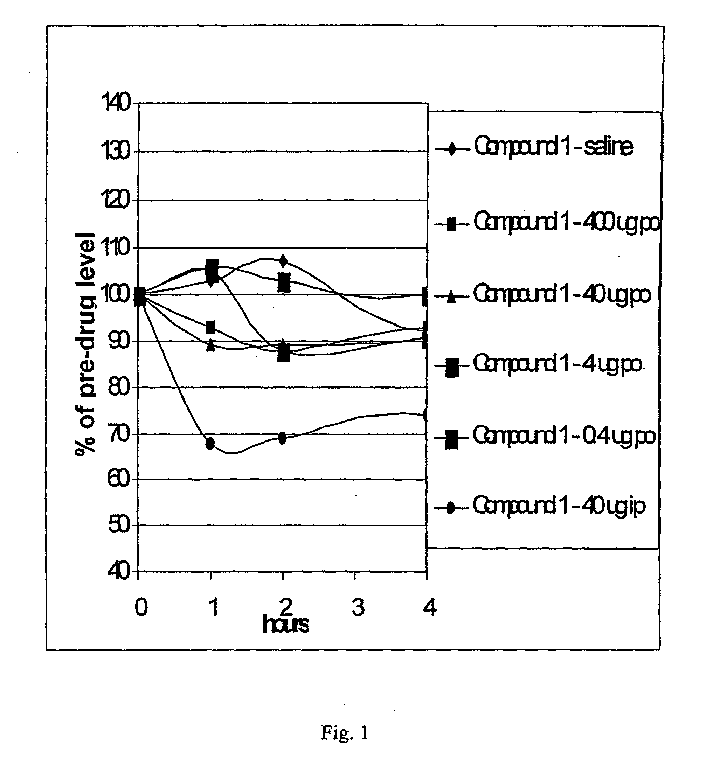

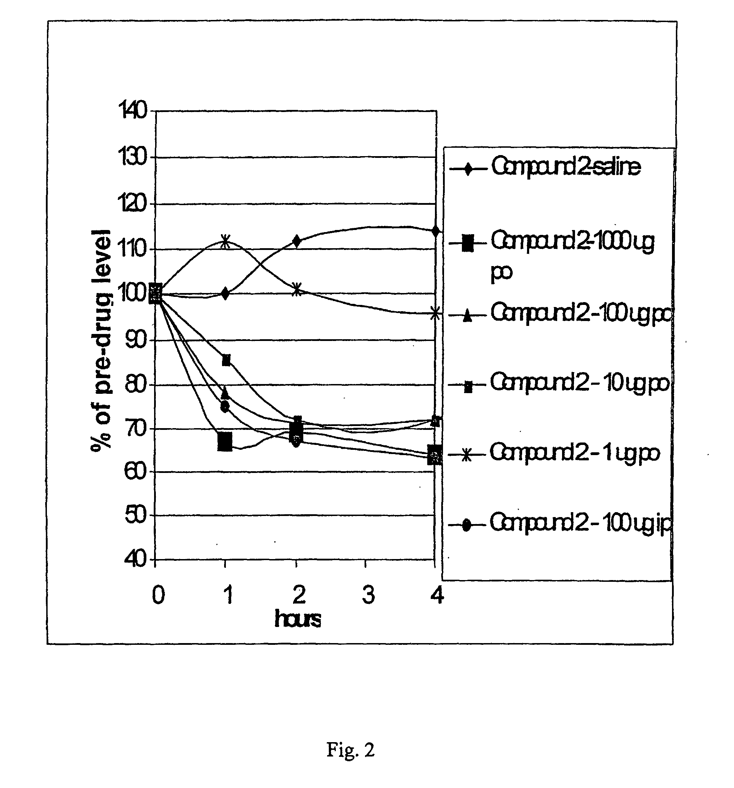

The results obtained are shown in Table 3 and in Figures 1-2.

These results show that all tested compounds have an effect in lowering blood glucose levels. The effect is most pronounced when Compound 1 is given intraperitoneally whereas the effect of 1000µg po of Compound 2 is comparable to the effect of 100 µg ip of Compound 2. The potency of Compound 1 (des Pro³⁶-exendin-4(1-39)NH_{2,} SEQ ID NO:101) and Compound 2 (des Pro³⁶-exendin-4(1 -39)-Lys₆-NH_{2,} SEQ IDNO:93) when given intraperitoneally is shown to be very similar to exendin-4(1-39)-NH₂ (Compound (i)) itself(data not given) administered in the same way.

For Compound 1, des Pro³⁶-exendin-4(1-39)-NH₂ (SEQ ID NO:101), there is no effect in lowering blood glucose levels up to a dose of 400 µg/mouse when the compound is administered perorally, whereas for the same compound with the addition of the Lys6 fragment there is activity seen at a dose of 10 µg/mouse. This indicates that the minimum effective oral dose of the des Pro³⁶-exendin-4(1-39)-Lys₆-NH2 (SEQ ID NO:93) is at least 40 times lower than for des Pro³⁶-exendin-4(1-39)-NH₂ (SEQ ID NO:101).

These results show that the attachment of the sequence Z has no significant effect on the potency of the various peptides when administered interperitoneally while significantly enhancing the potency of the compound when administered perorally.

### 16. In vivo pharmacokinetics of Compound 1 and Compound 2 after i.v. administration to rabbits and pigs

In order to establish whether in vitro stability is sustained in vivo, the pharmacokinetic parameters of Compounds 1 and 2 was assessed in rabbits and using similar conditions in pigs.

### Chemicals and Reagents

Blank rabbit plasma in sodium heparin (5000 units/mL) were obtained from Harlan Sera Lab Ltd. (Loughborough, UK). OASIS^{TM} HLB solid phase extraction columns, 1 cc, 30 mg sorbent, were obtained from Waters (Milford, MA, USA) and ISOLUTE C18 (EC), 1 cc, SPE columns were obtained from IST (Mid Glamorgan, U.K.). The LC/MS analysis was performed on a HP 1100 instrument consisting of an on-line degasser, a binary gradient pump, an auto sampler, a column oven, Hewlett Packard (Wilmington, DE, USA) in combination with a Quattro Ultima mass spectrometer from Micromass (Altrincham, UK) both the LC and MS were controlled by MassLynx 33 software. The LC separations prior to MS detection were performed on a Vydac 218MS52 (2.1 x 250 mm) column (Hesperia, CA, USA).

### Drugs and dose levels:

The peptides were dissolved in pyrogen free isotonic saline and both peptides were administered iv. to rabbits and rats using a dose of 1000 nmol/kg.

### Rabbits:

Fifteen New Zealand White rabbits weighing 2.5 to 3.0 kg were used for the experiment. On the day of the experiment, the rabbits were anaesthetised with Hypnorm® i.m. followed by Dormicum® i.v.. Catheters were inserted into the femoral vein and artery for i.v. administration of drugs and arterial blood sampling. The rabbits stayed unconscious throughout the experiment.

### Sample Treatment:

Blood samples were collected at t = 1, 3, 5, 10, 15, 20, 30, 40, 60, 90, 120, 150, 180 ,and 240 min. The blood was collected in EDTA containing ice-chilled tubes and immediately centrifuged at 4 C for 5 min (20.000 x g). Plasma (250 µl) was transferred to ice-chilled 0.75 ml PLC vials containing 250 µl extraction solution (MeCN: 0.18 M Ammonium Carbonate pH 9.5, 10:90 v/v). The plasma samples were stored at -20 C until SPE and LC/MS analysis.

### Solid Phase Extraction:

The drug containing plasma samples (400 µl) are loaded onto OASIS^{TM} HLB (Compound 4) or ISOLUTE^{TM} (Compound (iii)) solid phase extraction columns preconditioned with 950 µl MeCN followed by 950 µl water. The columns are washed with 950 µl 2 % TFA in water followed by an equal volume of 2 % TFA in MeCN:water (20:78 v/v). The analytes are eluted with 500 µl 2 % TFA in MeCN:water (60:38 v/v) and analysed by LC/MS.

### LC/MS

The samples were kept at 18 C in the auto sampler tray prior to injection of 20 to 50 µl onto the LC column (Vydac 218MS52 (2.1 x 250 mm). The separations were performed at 30°C using a flow rate of 250 µl/min and a gradient according to the table below. Both the test compound and the reference drug are detected by single ion recording (SIR) using the m/z = 676.7 and the m/z= 1095.2 and 821.8 ion species, respectively. All instrument conditions were controlled by MassLynx software ver. 3.3 software.

The plasma samples were analysed and the plasma concentration (Cₚₗ) plotted versus time in a semi log diagram. The plasma concentration were followed for three hours in rabbits. The Cₚₗ vs. time curves from the individual rabbits were fitted to a two-compartment open model (figure not shown) using 1/y² weighted least squares in WinNonlin 3.1 (Pharsight Corp. (Mountain View, CA)). The pharmacokinetic constants obtained from the data analysis are listed in Table 4.

**Table 4**

| In vivo kinetics in rabbits and pigs ** | | | |
|---|---|---|---|
| Parameter | Comp. 1 (n=5) Mean | Comp. 2 (n=5) Mean | Comp. 2 ** (n=2) Mean |
| T_{½}, α min | 4.4 | 11 | 16 |
| T_{½}, β min | 23 | 69 | 252 |

Table 4: The pharmacokinetic constants were obtained from rabbits when the Cₚₗ vs. time curves was fitted mathematically. The compounds were administered iv in a concentration of 1000 nmol/kg. T_{1/2} values are given in minutes (min) for the α and β phase. Statistics: two-tailed t-test assuming samples with unequal variances showed p<0.001 for all measured parameters. In conclusion the T1/2 value for Compound 2 is approximately three times the value calculated for Compound 1 which represents the unconjugated equivalent.

### 17. Glucose tolerance test of Compounds 2, 14-16, 18 and 19 compared to Compound (i)

Male diabetic db/db mice (M&B, Bomholdtgaard, Ll. Skensved, Denmark) are used. This well-described mouse model has inherited malfunctions of the glucose metabolism due to a mutation in the leptin receptor. Like human patients with uncontrolled non-insulin demanding diabetes mellitus (NIDDM), homozygous db/db mice experience polydipsia, polyuria and glycosuria and gain weight during their first 3 months of life despite their hyperglycaemic stage. However, in this model the hyperglycaemia is associated with progressive pancreatic islet atrophy with possible ketosis and death at 6-8 months of age. Thus, attention should be paid to the progression and status of their disease state. Therefore, preferably only db/db mice less than 16 weeks old should be used for drug testing of GLP-1 analogues.

All animals are acclimatised for at least one week and handled daily for two days prior to the first oral glucose tolerance test (OGTT). Furthermore, to reduce stress-induced glucose excursions, the animals should be subjected to at least one OGTT without compound as described below prior to the experiment. Due to the great scatter of glucose tolerance among diabetic mice, the animals are stratified by an OGTT prior to their first use.

### Peptides

Peptides are dissolved in 0.1 M phosphate-buffered saline (PBS) with 0.1% bovine albumin where pH is adjusted to 7.4 by adding 5 M NaOH. All solutions are prepared fresh on the morning immediately before the experiment. Vehicle treated animals are given PBS with 0.1% albumin alone.

### Glucose Tolerance Test and Dosing

Before the oral glucose tolerance test, the animals are fasted for 17 hours (from 4 p.m. until 9 a.m. the following morning). Beginning at 9.00 a.m. blood is taken from the tail tip (t = -15 min) and blood glucose is measured. The whole blood glucose (mM) concentration is analysed by the immobilised glucose oxidase method using a drop of blood (< 5 µl. Elite Autoanalyser, Bayer, Denmark) following the manufacturer's manual. Animals with severe diabetes (> 10 mM) are excluded. Immediately after the initial blood sample, the animals receive an intraperitoneal (i.p.) injection of vehicle or a dose of antidiabetic compound. Injection volume is 200 µl/50 g body weight in all groups. Fifteen minutes after i.p. administration of the substance an oral dose of 1 g/kg glucose (Sigma, St. Louis) dissolved in water (200 µl/50 g body weight) is given, and the animals are returned to their home cages (t = 0). Blood glucose levels are measured at t=30 min, t=60 min, t=120 min and t=240 min. The animals are fasted during the observation period. For each animal a data log sheet was filled in at the time of each blood sampling.

### Calculations and Statistics

In order to analyse the effects of the compounds, the absolute and the relative difference from baseline (t=0) are calculated for each time point. The area under the curve for the whole experiment (AUC 0-240 min) is determined using the trapezoid method. On the day of stratification, the mice are distributed in order to ensure that the glucose tolerances are similar in all groups. However, to correct for the progression of the diabetes with time, a vehicle treated control group is tested on each day of experiment and the response to drugs are expressed relative to response observed in vehicle-treated time-control animals.

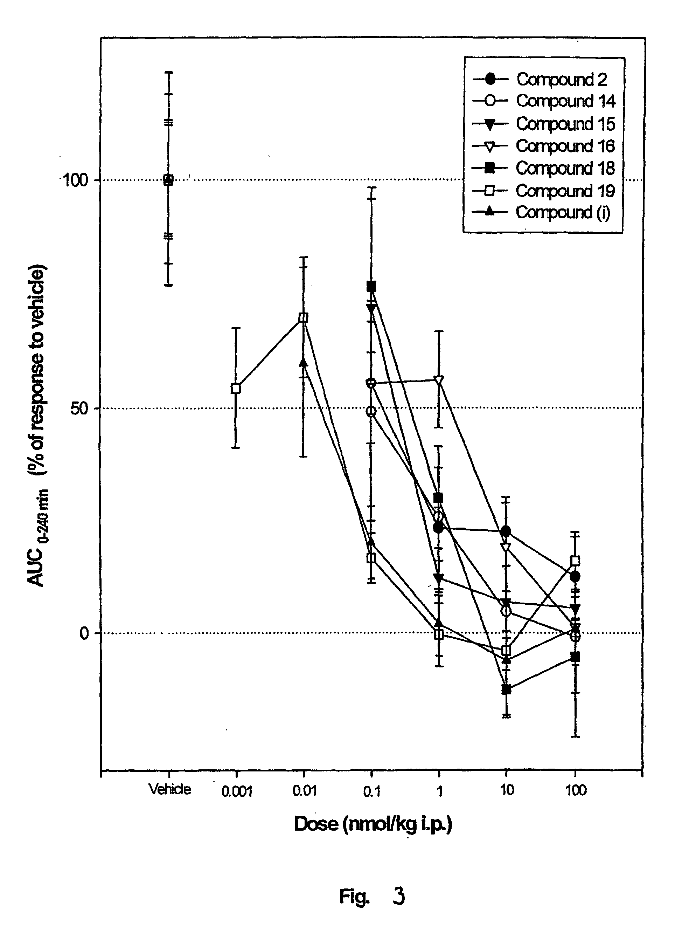

Dose-response curves for each substance are plotted, cf. Fig. 3, and the effect of drug relative to responses obtained during treatment with vehicle are analysed using an ANCOVA analysis (analysis of covariance). Treatment (drug or vehicle) is considered the independent variable, AUC 0-240 min expressed as per cent response in vehicle-treated time-control mice is the dependent variable, and drug dose is defined as covariate. Post-hoc analysis is performed using Fisher's Least Significant test. Differences are considered significant at the 0.05 level. Statistical analyses were performed using Statistica version 5.5 for Windows NT, StatSoft, Tulsa, Oklahoma, U.S A.. The dose response curves shown in Fig.3 clearly shows that all tested compounds exhibit a glucose lowering effect comparable to that of the reference drug.

### 18. Effects of Compound 2 and Compound (i) on OGGT in db/db mice

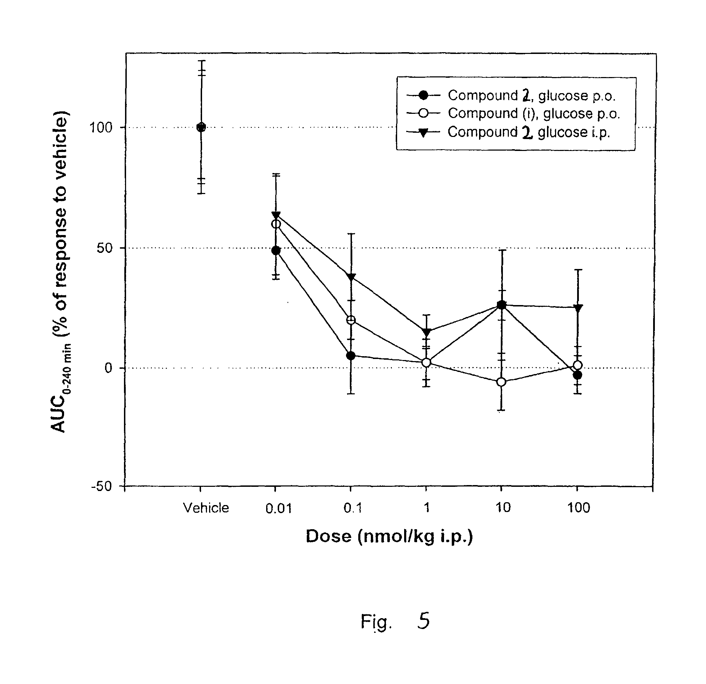

Figure 5 is a plot of AUC for Compound 2 and Compound (i) in an OGTT performed using the same experimental conditions as described in Example 17. The figure shows that the blood glucose lowering effect of Compound 2 is the same as the effect of the prior art compound (i).

### 19. Long term effects of Compound 2, 100 nmol/kg i.p. on the oral glucose tolerance test. (OGTT) when administered up to 24 hours before the OGTT

This experiment uses the maximal dose of 100 nmol/kg i.p. in db/db mice and otherwise the same experimental conditions as described in Example 17 are used. Results are shown in Fig. 6 and the conclusion of the experiment is that the duration of action of Compound 2 is up to 18 hours in db/db mice.

The invention described and claimed herein is not to be limited in scope by the specific embodiments herein disclosed, since these embodiments are intended as illustrations of several aspects of the invention. Any equivalent embodiments are intended to be within the scope of this invention. Indeed, various modifications of the invention in addition to those shown and described herein will become apparent to those skilled in the art from the foregoing description. Such modifications are also intended to fall within the scope of the appended claims.

## Claims

1. A peptide conjugate of an exendin-4(1-39) variant comprising a deletion of between 1 and 5 amino acid residues at positions corresponding to positions 34-38 of exendin-4, wherein the exendin-4 variant is coupled via its C-terminus to an amino acid sequence Z, where Z comprises from 1 to 7 Lys amino acid residues.

2. The peptide conjugate of claim 1, wherein the variant comprises a deletion of between 1 and 3 amino acid residues at positions corresponding to positions 36-38 of extendin 4.

3. The peptide conjugate of claim 1 or claim 2, wherein the variant comprises a deletion of 1, 2, or 3 Pro residues at positions corresponding to positions 36-38 of exendin-4.

4. The peptide conjugate of any one of claims 1 to 3, wherein Z is Lys₆.

5. The peptide conjugate of any one of the preceding claims, wherein the exendin-4 variant is des Pro³⁶ exendin-4 (1-39).

6. The peptide conjugate of any one of the preceding claims, wherein the peptide conjugate is in the form of a free acid or a pharmaceutically acceptable salt.

7. The peptide conjugate of any one of the preceding claims which is:
des Pro³⁶-exendin-4(1-39)-Lys₆-NH₂ (SEQ ID NO:93), des Pro³⁶Pro³⁷-exendin-4(1-39)-Lys₆-NH₂ or des Pro³⁶Pro³⁷Pro³⁸-exendin-4(1-39)-Lys₆-NH₂,or a free acid or a pharmaceutically acceptable salt thereof.

8. The peptide conjugate of claim 7 which is des Pro³⁶-exendin-4(1-39)-Lys₆-NH₂ (SEQ ID NO:93) or a free acid or a pharmaceutically acceptable salt thereof.

9. A pharmaceutical composition comprising the peptide conjugate of any one the preceding claims and a pharmaceutical acceptable carrier.

10. A peptide conjugate of any one of claims 1 to 8 for use in a method of medical treatment.

11. Use of the peptide conjugate of any one of claims 1 to 8 for the manufacture of a pharmaceutical composition.

12. Use of the peptide conjugate of any one of claims 1 to 8 for the preparation of a medicament for the treatment of type 1 or type 2 diabetes, insulin resistance syndrome, obesity, eating disorders, hyperglycemia, metabolic disorders or gastric disease.

## Patentansprüche

1. Peptidkonjugat einer Exendin-4(1-39)-Variante, die eine Deletion von zwischen 1 und 5 Aminosäureresten an Positionen aufweist, die den Positionen 34 bis 38 von Exedin-4 entsprechen, worin die Exendin-4-Variante über ihren C-Terminus an eine Aminosäuresequenz Z gekuppelt ist, worin Z 1 bis 7 Lys-Aminosäurereste umfasst.

2. Peptidkonjugat nach Anspruch 1, worin die Variante eine Deletion von zwischen 1 und 3 Aminosäureresten an Positionen umfasst, die den Positionen 36 bis 38 von Exendin 4 entsprechen.

3. Peptidkonjugat nach Anspruch 1 oder 2, worin die Variante eine Deletion von 1, 2 oder 3 Pro-Resten an Positionen umfasst, die den Positionen 36 bis 38 von Exendin-4 entsprechen.

4. Peptidkonjugat nach einem der Ansprüche 1 bis 3, worin Z Lys₆ ist.

5. Peptidkonjugat nach einem der vorangegangenen Ansprüche, worin die Exendin-4-Variante des-Pro³⁶-Exendin-4(1-39) ist.

6. Peptidkonjugat nach einem der vorangegangenen Ansprüche, worin das Peptidkonjugat in Form einer freien Säure oder eines pharmazeutisch annehmbaren Salzes vorliegt.

7. Peptidkonjugat nach einem der vorangegangenen Ansprüche, das Folgendes ist:
des-Pro³⁶-Exendin-4(1-39)-Lys₆-NH₂ (Seq.-ID Nr. 93), des-Pro³⁶Pro³⁷-Exendin-4(1-39)-Lys₆-NH₂ oder des-Pro³⁶Pro³⁷Pro³⁸-Exendin-4(1-39)-Lys₆-NH₂ oder eine freie Säure oder ein pharmazeutisch annehmbares Salz davon.

8. Peptidkonjugat nach Anspruch 7, das des-Pro³⁶-Exendin-4(1-39)-Lys₆-NH₂ (Seq.-ID Nr. 93) oder eine freie Säure oder ein pharmazeutisch annehmbares Salz davon ist.

9. Pharmazeutische Zusammensetzung, die ein Peptidkonjugat nach einem der vorangegangenen Ansprüche und einen pharmazeutisch annehmbaren Träger umfasst.

10. Peptidkonjugat nach einem der Ansprüche 1 bis 8 zur Verwendung in einem medizinischen Behandlungsverfahren.

11. Verwendung eines Peptidkonjugats nach einem der Ansprüche 1 bis 8 zur Herstellung einer pharmazeutischen Zusammensetzung.

12. Verwendung eines Peptidkonjugats nach einem der Ansprüche 1 bis 8 zur Herstellung eines Medikaments zur Behandlung von Typ 1- oder Typ 2-Diabetes, Insulinresistenzsyndrom, Fettleibigkeit, Essstörungen, Hyperglykämie, Stoffwechselstörungen oder Magenerkrankungen.

## Revendications

1. Conjugué de peptide d'une variante d'exendine-4(1-39) comprenant une délétion ayant entre 1 et 5 résidus d'acides aminés en des positions correspondant aux positions 34-38 de l'exendine-4 où la variante d'exendine-4 est couplée via son C-terminal à une séquence d'acides aminés Z où Z comprend de 1 à 7 résidus d'acides aminés Lys.

2. Conjugué de peptide de la revendication 1, où la variante comprend une délétion ayant entre 1 et 3 résidus d'acides aminés en des positions correspondant aux positions 36-38 de l'extendine-4.

3. Conjugué de peptide de la revendication 1 ou la revendication 2, où la variante comprend une délétion de 1, 2 ou 3 résidus Pro en des positions correspondant aux positions 36-38 de l'exendine-4.

4. Conjugué de peptide de l'une quelconque des revendications 1 à 3, où Z est Lys₆.

5. Conjugué de peptide de l'une quelconque des revendications précédentes, où la variante d'exendine-4 est des Pro³⁶ exendine-4(1-39).

6. Conjugué de peptide de l'une quelconque des revendications précédentes où le conjugué de peptide est sous la forme d'un acide libre ou d'un sel pharmaceutiquement acceptable.

7. Conjugué de peptide de l'une quelconque des revendications précédentes qui est :
des Pro³⁶ -exendine-4(1-39)-Lys₆-NH₂(SEQ ID NO:93), des Pro³⁶ Pro³⁷-exendine-4(1-39)-Lys₆-NH₂ ou des Pro³⁶ Pro³⁷ Pro³⁸-exendine-4(1-39)-Lys₆-NH₂ ou un acide libre ou un sel pharmaceutiquement acceptable.

8. Conjugué de peptide de la revendication 7 qui est des Pro -exendine-4(1-39)-Lys₆-NH₂ (SEQ ID NO:93) ou un acide libre ou un sel pharmaceutiquement acceptable.

9. Composition pharmaceutique comprenant le conjugué de peptide de l'une quelconque des revendications précédentes et un support pharmaceutiquement acceptable.

10. Conjugué de peptide de l'une quelconque des revendications 1 à 8 à utiliser dans une méthode de traitement médical.

11. Utilisation du conjugué de peptide de l'une quelconque des revendications 1 à 8 pour la fabrication d'une composition pharmaceutique.

12. Utilisation du conjugué de peptide de l'une quelconque des revendications 1 à 8 pour la préparation d'un médicament pour le traitement du diabète type 1 ou 2, du syndrome de résistance à l'insuline, de l'obésité, des troubles de nutrition, de l'hyperglycémie, des troubles métaboliques ou d'une maladie gastrique.
